(19) Europäisches Patentamt
European Patent Office
Office européen des brevets

(11) Veröffentlichungsnummer: **0 368 176**
**A1**

(12) # EUROPÄISCHE PATENTANMELDUNG

(21) Anmeldenummer: **89120426.5**

(51) Int. Cl.5: **C07D 223/18, A01N 47/42**

(22) Anmeldetag: **04.11.89**

Patentanspruch für folgenden Vertragsstaat: ES

(30) Priorität: **11.11.88 CH 4191/88**
**24.08.89 CH 3067/89**

(43) Veröffentlichungstag der Anmeldung:
**16.05.90 Patentblatt 90/20**

(84) Benannte Vertragsstaaten:
**AT BE CH DE ES FR GB GR IT LI LU NL SE**

(71) Anmelder: **F. HOFFMANN-LA ROCHE AG**
**Postfach 3255**
**CH-4002 Basel(CH)**

(72) Erfinder: **Zurflüh, René, Dr.**
**Dachslenbergstrasse 54**
**CH-8180 Bülach(CH)**

(74) Vertreter: **Urech, Peter, Dr. et al**
**Grenzacherstrasse 124 Postfach 3255**
**CH-4002 Basel(CH)**

(54) **Carboximidsäureester, deren Herstellung und deren Verwendung als Schädlingsbekämpfungsmittel.**

(57) Die Erfindung betrifft neue Verbindungen der Formel

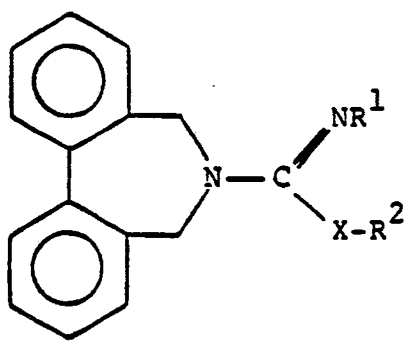

I

worin $R^1$, $R^2$ und X die in der Beschreibung angegebenen Bedeutungen besitzen, deren Säureadditionssalze und die Herstellung dieser Substanzen, Schädlingsbekämpfungsmittel, die solche Substanzen als Wirkstoffe enthalten, und die Verwendung der Wirkstoffe bzw. Mittel zur Schädlingsbekämpfung.

EP 0 368 176 A1

## Carboximidsäureester, deren Herstellung und deren Verwendung als Schädlingsbekämpfungsmittel

Die vorliegende Erfindung betrifft heterocyclische Verbindungen, und zwar 5,7-Dihydro-6H-dibenz[c,e]-azepin-6-(thio)carboximidsäureester der allgemeinen Formel

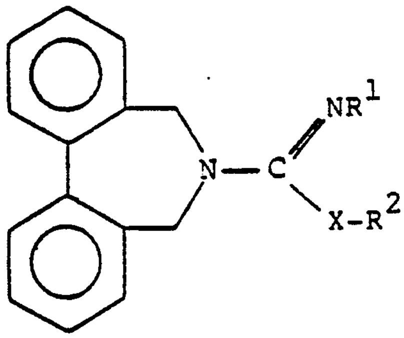

I

worin

$R^1$ Wasserstoff oder eine Gruppe -Y-$R^3$ ist,

$R^2$ mit $C_{3-6}$-Cycloalkyl, Aryl, Aryloxy oder Heteroaryl substituiertes $C_{1-6}$-Alkyl, $C_{3-6}$-Alkenyl oder $C_{3-6}$-Alkinyl ist, wobei im Falle von $R^1$ = -Y-$R^3$ die Substitution fakultativ ist,

oder

$R^2$ gegebenenfalls mit Aryl oder Aryloxy substituiertes, durch ein oder zwei Sauerstoffatome unterbrochenes $C_{2-6}$-Alkyl ist,

oder

$R^2$ Aryl, Heteroaryl, 2-Tetrahydrofuranylmethyl, 2-Tetrahydropyranylmethyl oder eine der Gruppen (a) bis (c)

$-N=CR^4R^5$ (a)

$-Z-ON=CR^4R^5$ (b)

$-Z-NR^6R^7$ (c)

ist,

$R^3$ Wasserstoff; gegebenenfalls substituiertes $C_{1-6}$-Alkyl, $C_{2-6}$-Alkenyl, $C_{2-6}$-Alkinyl, $C_{3-6}$-Cycloalkyl, Phenyl, Naphthyl, p-Biphenylyl, Benzylphenyl, Phenoxyphenyl, Benzoylphenyl, 3,4-Methylendioxyphenyl oder fünf- bis sechs-gliedriges Heterocyclyl; eine Gruppe $-OR^8$ (d); oder eine Gruppe $-NR^9R^{10}$ (e),

$R^4$ $C_{1-6}$-Alkyl,

$R^5$ $C_{1-6}$-Alkyl oder Phenyl,

oder

$R^4$ und $R^5$ zusammen Tetra-, Penta- oder Hexamethylen,

$R^6$ Wasserstoff oder $C_{1-4}$-Alkyl,

$R^7$ $C_{1-4}$-Alkyl, $C_{2-5}$-Alkanoyl oder $C_{2-5}$-Alkoxycarbonyl,

oder

$R^6$ und $R^7$ zusammen mit dem Stickstoffatom, an das sie gebunden sind, eine gegebenenfalls mit einem oder zwei $C_{1-4}$-Alkylresten substituierte fünf- bis siebengliedrige heterocyclische Gruppe, die neben dem bereits erwähnten Stickstoffatom auch noch 1 oder 2 aus Sauerstoff, Schwefel und Stickstoff ausgewählte Heteroatome und/oder eine Ketofunktion im Ring aufweisen kann,

$R^8$ $C_{1-6}$-Alkyl, $C_{2-6}$-Halogenalkyl, $C_{3-6}$-Alkenyl, $C_{3-6}$-Alkinyl, Benzyl oder gegebenenfalls substituiertes Phenyl,

$R^9$ Wasserstoff oder $C_{1-4}$-Alkyl,

$R^{10}$ $C_{1-6}$-Alkyl oder gegebenenfalls substituiertes Phenyl,

X Sauerstoff oder Schwefel, wobei, falls $R^2$ eine Gruppe (a) ist, nur Sauerstoff,

Y Carbonyl, Sulfinyl oder Sulfonyl, wobei, falls $R^3$ Wasserstoff oder eine Gruppe (d) oder (e) ist, nur Carbonyl,

und

Z $C_{1-4}$-Alkylen bedeuten,

sowie Säureadditionssalze derjenigen Verbindungen I, in denen $R^1$ Wasserstoff bedeutet.

Die erfindungsgemässen Verbindungen, d.h. die Verbindungen der Formel I und die oben angesprochenen Säureadditionssalze, sind Schädlingsbekämpfungsmittel und eignen sich insbesondere zur Bekämpfung von Insekten, Nematoden und Milben, z.B. Spinnmilben. Somit umfasst die Erfindung auch Schädlingsbekämpfungsmittel, welche erfindungsgemässe Verbindungen als Wirkstoffe enthalten, ein Verfahren zur Herstellung dieser Verbindungen sowie die Verwendung dieser Verbindungen bzw. Mittel zur Bekämpfung von Schädlingen.

Sämtliche Gruppierungen "Alkyl", "Alkenyl", "Alkinyl" und "Alkylen" können je nach Anzahl deren Kohlenstoffatome geradkettig oder verzweigt sein. Zudem können die Alkenyl- und Alkinylreste jeweils mehr als eine Doppel- bzw. Dreifachbindung aufweisen. Ein allfällig vorhandenes Halogen atom ist Fluor, Chlor, Brom oder Jod. Eine Gruppe, wie beispielsweise Alkyl, Alkenyl, Alkinyl, Aryl oder Heteroaryl als solches oder als Teil einer grösseren Gruppe, die jeweils mehr als zwei Halogensubstituenten trägt, kann gleiche oder verschiedene Halogenatome aufweisen. Unter Aryl sind insbesondere Phenyl und Naphthyl, unter Heteroaryl insbesondere heterocyclische Gruppen mit aromatischem Charakter, wie Pyridyl, Furyl, Thienyl sowie solche Gruppen mit ankondensiertem Benzol, z.B. Chinolinyl und Chinoxalinyl, zu verstehen. Dies gilt auch für Aryl als Teil einer grösseren Gruppe, z.B. Aryloxy. Die Aryl-, Aryloxy- und Heteroarylgruppen können jeweils einen oder mehrere Substituenten aufweisen. Im Falle von Aryl, Aryloxy oder Heteroaryl als Substituenten eines $C_{1-6}$-Alkyl-, $C_{3-6}$-Alkenyl- oder $C_{3-6}$-Alkinylrestes ($R^2$) sind die Substituenten geeigneterweise einer oder zwei aus Halogenatomen und $C_{1-6}$-Alkyl-, $C_{1-4}$-Halogenalkyl-, $C_{1-4}$-Alkoxy-, $C_{1-4}$-Halogenalkoxy-, $C_{1-4}$-Alkylthio-, $C_{1-4}$-Halogenalkylthio-, Nitro-, Cyano-, Phenyl- und Phenoxygruppen ausgewählte Substituenten. Bedeutet $R^2$ Aryl oder Heteroaryl und falls dies einen oder mehrere Substituenten aufweist, sind die Substituenten geeigneterweise einer oder zwei aus Halogenatomen und $C_{1-4}$-Alkyl-, $C_{1-4}$-Halogenalkyl-, $C_{1-4}$-Alkoxy-, $C_{1-4}$-Halogenalkoxy-, $C_{1-4}$-Alkylthio-, $C_{1-4}$-Halogenalkylthio-, Nitro- und Cyanogruppen ausgewählte Substituenten. Es kommen als Substituenten für $C_{3-6}$-Cycloalkyl ($R^2$ und $R^3$) insbesondere bis 4 aus Halogen, $C_{1-4}$-Alkyl und/oder $C_{1-4}$-Alkoxy ausgewählte Substituenten in Frage. In diesem Fall ist ein allfällig vorhandener Halogensubstituent vorzugsweise Fluor oder Chlor. Falls $R^3$ substituiertes $C_{1-6}$-Alkyl, $C_{2-6}$-Alkenyl oder $C_{2-6}$-Alkinyl bedeutet, ist der Alkyl-, Alkenyl oder Alkinylrest geeigneterweise ein- oder mehrfach mit Halogen, $C_{1-4}$-Alkoxy, Cyano, gegebenenfalls substituiertem Phenyl und/oder gegebenenfalls substituiertem Phenoxy substituiert. Bei dem gleich erwähnten gegebenenfalls substituierten Phenyl oder Phenoxy sowie bei dem als Bedeutung von $R^3$ selbst vorgesehenen gegebenenfalls substituierten Phenyl, Naphthyl, p-Biphenylyl, Benzylphenyl, Phenoxyphenyl, Benzoylphenyl oder 3,4-Methylendioxyphenyl handelt es sich um eine solche aromatische Gruppe, die unsubstituiert oder ein- bis dreifach mit Halogen, $C_{1-4}$-Alkyl, $C_{1-4}$-Halogenalkyl, $C_{1-4}$-Alkoxy, $C_{1-4}$-Halogenalkoxy, $C_{1-4}$-Alkylthio, $C_{1-4}$-Halogenalkylthio, Nitro, Cyano, Amino, $C_{1-4}$-Alkylamino, Di($C_{1-4}$-alkyl)amino, Carboxy und/oder $C_{2-5}$-Alkoxycarbonyl substituiert ist. Dies gilt auch für $R^8$ und $R^{10}$ als gegebenenfalls substituiertes Phenyl. Unter fünf- bis sechs-gliedrigem Heterocyclyl ($R^3$) sind solche heterocyclischen Gruppen zu verstehen, die neben Ring-Kohlenstoffatomen 1 bis 3 aus Stickstoff-, Sauerstoff- und Schwefelatomen ausgewählte Heteroatome im Ring aufweisen und aromatisch, teilweise gesättigt oder ganz gesättigt sind; auch heterocyclische Gruppen mit ankondensiertem Benzol kommen in Frage. Diese heterocyclischen Gruppen sind gegebenenfalls mit 1 bis 2 Halogenatomen, 1 Cyanogruppe, 1 $C_{1-4}$-Alkylrest, 1 $C_{1-4}$-Halogenalkylrest und/oder 1 $C_{1-4}$-Alkoxygruppe substituiert, wobei im Falle des Vorhandenseins eines ankondensierten Benzolrings dieser seinerseits mit einem oder mehreren aus Halogen, Methyl, Methoxy und Trifluormethyl ausgewählten Substituenten substituiert sein kann. Beispiele solcher Heterocyclylgruppen sind Pyridyl, Pyrrolyl, Piperidinyl, Pyrazinyl, Imidazolyl, 1,2,4-Triazolyl, Furyl, Thiophenyl, Morpholinyl, Chinolinyl und Benzofuryl.

Falls in den Verbindungen der Formel I asymmetrische Kohlenstoffatome vorliegen, treten die Verbindungen in optisch aktiver Form auf. Im Falle derjenigen Verbindungen der Formel I, in denen aliphatische Doppelbindungen vorliegen, kann zusätzlich geometrische Isomere auftreten. Auf jeden Fall treten die Verbindungen I allein aufgrund des Vorhandenseins der Imino-Doppelbindung in der [E]- oder [Z]-Form auf. Ferner kann Atropisomerie auftreten. Die Formel I soll demnach all diese möglichen isomeren Formen sowie deren Gemische, z.B. racemische Gemische und beliebige [E/Z]-Gemische, umfassen.

Als Säureadditionssalze der Verbindungen der Formel I kommen physiologisch verträgliche Salze in Frage. Hierzu gehören Salze dieser Verbindungen mit anorganischen und organischen Säuren, vorzugsweise Halogenwasserstoffsäuren, wie Salzsäure und Bromwasserstoffsäure; Salpetersäure; Phosphorsäure; Schwefelsäure; mono- und bifunktionellen Carbonsäuren und Hydroxycarbonsäuren, wie Essigsäure, Maleinsäure, Bernsteinsäure, Fumarsäure, Weinsäure, Zitronensäure, Salicylsäure, Sorbinsäure und Milchsäure; und Sulfonsäure, wie 1,5-Naphthalin-disulfonsäure.

Eine besondere Gruppe von Verbindungen der Formel I besteht aus denjenigen Verbindungen der Formel I, in der $R^1$, $R^2$ und X die oben angegebenen Bedeutungen besitzen und $R^3$ Wasserstoff; gegebenenfalls substituiertes $C_{1-6}$-Alkyl, $C_{3-6}$-Alkenyl, $C_{3-6}$-Alkinyl, $C_{3-6}$-Cycloalkyl, Phenyl, Naphthyl, p-Biphenyl, Benzylphenyl, Phenoxyphenyl, Benzoylphenyl oder fünf- bis sechs-gliedriges Heterocyclyl; eine Gruppe $-OR^8$ (d), in der $R^8$ $C_{1-6}$-Alkyl oder gegebenenfalls substituiertes Phenyl ist; oder eine Gruppe $-NR^9R^{10}$, in der $R^9$ und $R^{10}$ die oben angegebenen Bedeutungen besitzen, bedeuten.

Unabhängig voneinander bedeuten $R^1$ vorzugsweise eine Gruppe $-Y-R^3$ und X vorzugsweise Sauerstoff. Im Falle der Verbindungen I, in denen $R^1$ Wasserstoff bedeutet, ist $R^2$ vorzugsweise eine Gruppe (b) oder (c), in der Z Aethylen bedeutet, während in den Verbindungen I, in denen $R^1$ eine Gruppe $-Y-R^3$ bedeutet, $R^2$ vorzugsweise $C_{1-6}$-Alkyl, insbesondere $C_{1-4}$-Alkyl, und unabhängig davon $R^3$ vorzugsweise gegebenenfalls substituiertes Phenyl, gegebenenfalls substituiertes fünf- bis sechs-gliedriges Heterocyclyl oder eine Gruppe (d) und Y vorzugsweise Carbonyl bedeuten.

Bevorzugte einzelne Verbindungen der Formel I sind:
5,7-Dihydro-6H-dibenz[c,e]azepin-6-carboximidsäure-{2-[(isopropylidenamino)oxy]-äthyl}ester,
5,7-Dihydro-6H-dibenz[c,e]azepin-6-carboximidsäure-(2-athoxycarbonylamino-äthyl)ester,
N-Benzoyl-5,7-dihydro-6H-dibenz[c,e]azepin-6-carboximidsäure-äthylester,
N-(p-Chlorbenzoyl)-5,7-dihydro-6H-dibenz[c,e]azepin-6-carboximidsäure-äthylester,
N-Isonicotinoyl-5,7-dihydro-6H-dibenz[c,e]azepin-6-carboximidsäure-äthylester,
N-Aethoxycarbonyl-5,7-dihydro-6H-dibenz[c,e]azepin-6-carboximidsäure-äthylester,
N-(1-Imidazolylcarbonyl)-5,7-dihydro-6H-dibenz[c,e]azepin-6-carboximidsäure-äthylester,
N-(p-Brombenzoyl)-5,7-dihydro-6H-dibenz[c,e]azepin-6-carboximidsäure-äthylester,
N-(p-tert.Butylbenzoyl)-5,7-dihydro-6H-dibenz[c,e]azepin-6-carboximidsäure-äthylester,
N-(p-Fluorbenzoyl)-5,7-dihydro-6H-dibenz[c,e]azepin-6-carboximidsäure-äthylester,
N-Methoxycarbonyl-5,7-dihydro-6H-dibenz[c,e]azepin-6-carboximidsäure-äthylester,
5,7-Dihydro-6H-dibenz[c,e]azepin-6-carboximidsäure-cyclopropylmethylester,
N-(p-Trifluormethylbenzoyl)-5,7-dihydro 6H-dibenz[c,e]azepin-6-carboximidsäure-äthylester,
N-(p-Anisoyl)-5,7-dihydro-6H-dibenz[c,e]azepin-6-carboximidsäure-äthylester,
N-Benzoyl-5,7-dihydro-6H-dibenz[c,e]azepin-6-carboximidsäure-methylester,
N-Benzoyl-5,7-dihydro-6H-dibenz[c,e]azepin-6-carboximidsäure-propylester,
N-(2-Furoyl)-5,7-dihydro-6H-dibenz[c,e]azepin-6-carboximidsäure-äthylester und
N-Cyclopropylcarbonyl-5,7-dihydro-6H-dibenz[c,e]azepin-6-carboximidsäure-äthylester,
aus denen die 1.-, 3.-, 6.-, 10.- und 11.- genannte Verbindung jeweils als besonders bevorzugt gilt.

Weitere Vertreter von Verbindungen der Formel I sind:
5,7-Dihydro-6H-dibenz[c,e]azepin-6-carboximidsäure-(2-furfuryl)ester,
5,7-Dihydro-6H-dibenz[c,e]azepin-6-carboximidsäure-[2-(2-phenoxyäthoxy)-äthyl]ester,
5,7-Dihydro-6H-dibenz[c,e]azepin-6-carboximidsäure-(3-pyridylmethyl)ester,
5,7-Dihydro-6H-dibenz[c,e]azepin-6-carboximidsäure-(2-piperidino-äthyl)ester,
5,7-Dihydro-6H-dibenz[c,e]azepin-6-carboximidsäure-{2-[(1-äthylpentylidenamino)oxy]-äthyl}ester,
5,7-Dihydro-6H-dibenz[c,e]azepin-6-carboximidsäure-{2-[(cyclohexylidenamino)oxy]-äthyl}ester,
5,7-Dihydro-6H-dibenz[c,e]azepin-6-carboximidsäure-[2-(N-methyl-methoxycarbonylamino)-äthyl]ester,
5,7-Dihydro-6H-dibenz[c,e]azepin-6-carboximidsäure-(2-methoxycarbonylamino-propyl)ester,
5,7-Dihydro-6H-dibenz[c,e]azepin-6-carboximidsäure-methoxycarbonylaminomethylester,
N-(2-Picolinoyl)-5,7-dihydro-6H-dibenz[c,e]azepin-6-carboximidsäure-äthylester,
N-Formyl-5,7-dihydro-6H-dibenz[c,e]azepin-6-carboximidsäure-äthylester,
N-Phenyläthinyl-5,7-dihydro-6H-dibenz[c,e]azepin-6-carboximidsäure-äthylester,
N-(1-Piperidincarbonyl)-5,7-dihydro-6H-dibenz[c,e]azepin-6-carboximidsäure-äthylester,
N-(1,2,4-Triazolyl-1-carbonyl)-5,7-dihydro-6H-dibenz[c,e]azepin-6-carboximidsäure-äthylester,
N-(o-Methoxybenzoyl)-5,7-dihydro-6H-dibenz[c,e]azepin-6-carboximidsäure-äthylester,
N-(p-Chlorbenzoyl)-5,7-dihydro-6H-dibenz[c,e]azepin-6-carboximidsäure-methylester,
N-(p-Chlorbenzoyl)-5,7-dihydro-6H-dibenz[c,e]azepin-6-carboximidsäure-propylester,
N-(p-Chlorbenzoyl)-5,7-dihydro-6H-dibenz[c,e]azepin-6-carboximidsäure-isopropylester,
N-(p-Jodbenzoyl)-5,7-dihydro-6H-dibenz[c,e]azepin-6-carboximidsäure-äthylester,
5,7-Dihydro-6H-dibenz[c,e]azepin-6-carboximidsäure-phenylester,
Cyclohexanon-O-[(5,7-dihydro-6H-dibenz[c,e]azepin-6-yl)carboximidoyl]oxim,
Acetophenon-O-[(5,7-dihydro-6H-dibenz[c,e]azepin-6-yl)carboximidoyl]oxim,
5,7-Dihydro-6H-dibenz[c,e]azepin-6-carboximidsäure-(2-dimethylaminoäthyl)ester,
5,7-Dihydro-6H-dibenz[c,e]azepin-6-carboximidsäure-(2-äthylcarbonylamino-äthyl)ester,
N-Propargyloxycarbonyl-5,7-dihydro-6H-dibenz[c,e]azepin-6-carboximidsäure-äthylester,
N-(2-Chlorisonicotinoyl)-5,7-dihydro-6H-dibenz[c,e]azepin-6-carboximidsäure-äthylester,
N-(2-Cyanoisonicotinoyl)-5,7-dihydro-6H-dibenz[c,e]azepin-6-carboximidsäure-äthylester,
N-(6-Chlornicotinoyl)-5,7-dihydro-6H-dibenz[c,e]azepin-6-carboximidsäure-äthylester,

N-(p-Benzylbenzoyl)-5,7-dihydro-6H-dibenz[c,e]azepin-6-carboximidsäure-äthylester,
N-(p-Benzoylbenzoyl)-5,7-dihydro-6H-dibenz[c,e]azepin-6-carboximidsäure-äthylester,
N-[p-(p-Chlorphenoxy)benzoyl]-5,7-dihydro-6H-dibenz[c,e]azepin-6-carboximidsäure-äthylester und
5,7-Dihydro-6H-dibenz[c,e]azepin-6-carboximidsäure-{2-[(1-methylisobutylidenamino)oxy]-äthyl}ester.

Das erfindungsgemässe Verfahren zur Herstellung der Verbindungen der Formel I und von ihren Säureadditionssalzen ist dadurch gekennzeichnet, dass man

a) zwecks Herstellung derjenigen Verbindungen der Formel I, in denen $R^1$ Wasserstoff bedeutet, 5,7-Dihydro-6H-dibenz[c,e]azepin -6-carbonitril, d.h. das Cyanamid der Formel

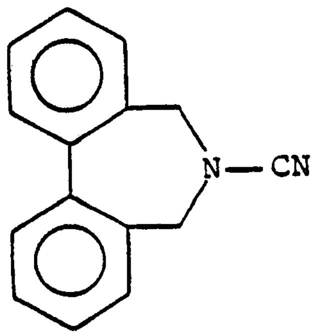

II

mit einem Alkohol bzw. Thiol der allgemeinen Formel

$R^{2'}$-$X'$H III

worin

$R^{2'}$ und $X'$ diejenigen oben angegebenen Bedeutungen von $R^2$ und X besitzen, die für den Zustand gelten, dass $R^1$ für Wasserstoff steht,

oder mit einem Alkalimetallsalz davon umsetzt, oder

b) zwecks Herstellung derjenigen Verbindungen der Formel I, in denen $R^1$ eine Gruppe -Y-$R^3$ bedeutet, ein Carboximidsäureester der allgemeinen Formel

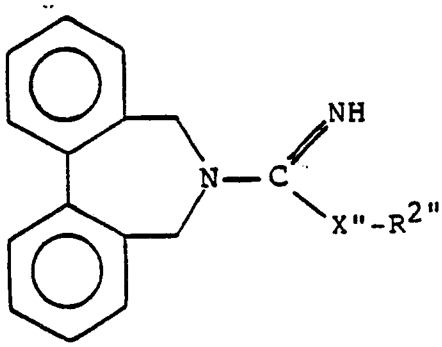

IV

worin

$R^{2''}$ und $X''$ diejenigen oben angegebenen Bedeutungen von $R^2$ und X besitzen, die für den Zustand gelten, dass $R^1$ für -Y-$R^3$ steht,

mit einer Carbonyl-, Sulfinyl- oder Sulfonylverbindung der allgemeinen Formel

Q-Y-$R^3$ V

worin

$R^3$ und Y die oben angegebenen Bedeutungen besitzen

und

Q eine Abgangsgruppe bedeutet,

umsetzt und erwünschtenfalls eine erhaltene Verbindung der Formel I, in der $R^1$ Wasserstoff bedeutet, durch Umsetzung mit einer Säure in das entsprechende Säureadditionssalz überführt.

Die Umsetzung nach Verfahrensvariante a) erfolgt zweckmässigerweise unter Verwendung überschüssigen Alkohols bzw. Thiols der Formel III als Lösungsmittel und in Gegenwart einer katalytischen oder stöchiometrischen Menge eines Alkalimetallsalzes, insbesondere des Natrium- oder Kaliumsalzes, des

Alkohols bzw. Thiols der Formel III. In einer weiteren Ausführungsform werden der Alkohol bzw. das Thiol der Formel III als Reaktionspartner und eine katalytische oder stöchiometrische Menge Alkalimetallcyanid, insbesondere Natrium- oder Kaliumcyanid, verwendet. Man kann auch noch ein Hilfslösungsmittel, wie einen aliphatischen Aether, z.B. Dimethoxyäthan oder tert.Butylmethyläther, oder einen gegebenenfalls halogenierten Aromaten, z.B. Toluol oder Chlorbenzol, verwenden. Die Reaktionstemperaturen können in einem grossen Bereich variiert werden und zwar im allgemeinen von 10 bis 120° C, vorzugsweise von 40 bis 100° C.

Die Umsetzung nach Verfahrensvariante b) stellt eine N-Acylierung, -Sulfinylierung bzw. -Sulfonylierung dar und kann unter den hierfür üblichen Reaktionsbedingungen durchgeführt werden. Als Verbindungen der Formel V eignen sich vorzugsweise die entsprechenden Halogenide, insbesondere Chloride (Q bedeutet Halogen bzw. Chlor), aber es kommen auch Säureanhydride [Q-Y-$R^3$ ist $O(COR^3)_2$], gemischte Säureanhydride, insbesondere diejenigen mit aliphatischen oder aromatischen Carbon- oder Sulfonsäuren [Q-Y-$R^3$ ist $O(Y'R^3)(Y''R^{11})$, worin $Y'$ und $Y''$ unabhängig voneinander Carbonyl oder Sulfonyl und $R^{11}$ eine aliphatische oder aromatische Gruppe bedeuten], Niederalkyl-, Benzyl- oder Arylester sowie Imidazolide in Frage.

Die Umsetzung erfolgt zweckmässigerweise in Gegenwart eines inerten organischen Verdünnungsmittels, wie eines aromatischen Kohlenwasserstoffes, z.B. Benzol oder Toluol; eines aliphatischen oder cyclischen Aethers, z.B. Diäthyläther, tert.Butylmethyläther, Tetrahydrofuran oder Dioxan; eines halogenierten aliphatischen Kohlenwasserstoffes, z.B. Methylenchlorid oder 1,2-Dichloräthan; Acetonitril; oder eines Dialkylamids, z.B. Dimethylformamid, bei Temperaturen zwischen -20° C und 100° C, vorzugsweise zwischen -10° C und 50° C.

In der Regel setzt man einen Carboximidsäureester IV mit einer Verbindung der Formel V in Gegenwart eines säurebindenden Mittels um, wie beispielsweise einer anorganischen Base, z.B. Kaliumcarbonat, oder einer organischen Base, z.B. Triäthylamin, Pyridin oder Chinolin, wobei jede der genannten organischen Basen gleichzeitig als Lösungsmittel dienen kann.

Sofern keine gezielte Synthese zur Isolierung reiner Isomerer durchgeführt wird, fällt das Produkt im Falle des Vorhandenseins zweier oder mehrerer Isomerer als Gemisch dieser Isomeren an. Die Isomeren können nach an sich bekannten Methoden aufgetrennt werden, oder sie können gewünschtenfalls auch z.B. durch Synthese aus entsprechenden optisch aktiven Ausgangsmaterialien hergestellt werden. E/Z-Isomerengemische können beispielsweise durch Chromatographie oder fraktionierte Kristallisation in die reinen Isomeren aufgetrennt werden.

Zur Herstellung der Säureadditionssalze der Verbindungen der Formel I werden die Verbindungen I mit den gewünschten Säuren auf übliche Weise umgesetzt, beispielsweise durch Lösen der Verbindung der Formel I in einem geeigneten Lösungsmittel und Hinzufügen der Säure.

Die Isolierung und die Reinigung der so hergestellten Verbindungen der Formel I bzw. der Säureadditionssalze können nach an sich bekannten Methoden erfolgen.

Das als Ausgangsmaterial in der Verfahrensvariante a) verwendete Cyanamid der Formel II ist z.B. aus der europäischen Patentpublikation Nr. 192.034 bekannt geworden. Bei den als Ausgangsmaterialien in der Verfahrensvariante b) verwendeten Carboximidsäureestern der Formel IV handelt es sich teilweise um welche, die ebenfalls aus der europäischen Patentpublikation Nr. 192.034 bekannt geworden sind, teilweise um welche, die gemäss der Verfahrensvariante a) herstellbar sind, also einen Teil der Verbindungen der Formel I darstellen. Die ferner als Ausgangsmaterialien verwendeten Verbindungen der Formeln III und V und die Alkalimetallsalze der ersteren sind zum grossen Teil bekannt oder können nach an sich bekannten Methoden hergestellt werden.

Die Verbindungen der Formel I und deren Säureadditionssalze sind ganz allgemein als Pestizide von Wert. Die erweisen sich als besonders wertvoll zur Bekämpfung von Milben, Insekten und Nematoden, insbesondere von

- Milben, die bei dem Pflanzenschutz von Bedeutung sind, wie z.B.

Tetranychidae (Spinnmilben), insbesondere Tetranychus urticae, Tetranychus cinnabarinus, Tetranychus turkestani, Tetranychus McDanieli, Tetranychus kanzawai;
Panonychus ulmi, Panonychus citri;
Phyllocoptruta oleivora;
Aculus schlechtendali;
Phyllocoptes vitis;
Aceria essigi, Aceria gracilis;
Cecidophyopsis ribis;
Eriophyes vitis, Eriophyes sheldoni, Eriophyes tulipae;
Eotetranychus sexmaculatus, Eotetranychus carpini;
Hemitarsonemus latus;

Acarus siro;
Bryobia graminum;
- Milben, die in der Veterinärmedizin von Bedeutung sind, wie z.B.
Macronyssus bursa, Macronyssus sylviarum, Macronyssus lacoti;
Dermanyssus gallinae;
Zecken, insbesondere der Familien Ixodidae und Argasidae und der Ordnungen Boophilus, Amblyomma, Hyalomma, Rhipicephalus, Ixodes, Argas und Ornithodorus.
- Nematoden, die bei dem Pflanzenschutz von Bedeutung sind, wie z.B.
Aphelenchoides sp., Globodera sp., Heliocotylenchus sp., Heterodera sp., Hoploliamus sp., Meloidogyne sp., Paratrichodorus sp., Pratylenchus sp., Rotylenchus sp., Tylenchorhynchus sp. und Tylenchulus sp.

Die erfindungsgemässen Verbindungen wirken als Kontakt- und Frassgifte. Zudem werden einige der Verbindungen von verschiedenen Pflanzen aufgenommen, so dass die zu bekämpfenden Schädlinge beim Fressen der Pflanzen vernichtet werden. Diese Verbindungen weisen also systemische Wirkung auf.

Es wirken die erfindungsgemässen Verbindungen auch gegen Spinnmilben, die gegenüber herkömmlichen Pestiziden Resistenz erworben haben. Die Verbindungen zeichnen sich ausserdem durch eine gute Residualwirkung und eine gute Selektivität gegenüber Phytoseiulus persimiles aus.

Das erfindungsgemässe Schädlingsbekämpfungsmittel ist dadurch gekennzeichnet, dass es eine wirksame Menge mindestens einer Verbindung der allgemeinen Formel I oder eines Säureadditionssalzes davon, wie oben definiert, sowie Formulierungshilfsstoffe enthält. Das Mittel enthält zweckmässigerweise zumindest einen der folgenden Formulierungshilfsstoffe:

Feste Trägerstoffe; Lösungs- bzw. Dispersionsmittel; Tenside (Netzmittel und Emulgatoren); Dispergiermittel (ohne Tensidwirkung); und Stabilisatoren.

Unter Verwendung dieser und gegebenenfalls zusätzlicher Hilfsstoffe können die Verbindungen der Formel I und ihre Säureadditionssalze, also die pestiziden Wirkstoffe, in die üblichen Formulierungen übergeführt werden, wie Lösungen, Suspensionen, Emulsionen, emulgierbare Konzentrate, Pasten, Schäume, Stäube, Pulver und Granulate.

Als feste Trägerstoffe kommen im wesentlichen in Frage: natürliche Mineralstoffe, wie Kaolin, Tonerden, Kieselgur, Talkum, Bentonit, Kreide, Kalkstein, Quarz, Dolomit, Attapulgit, Montmorillonit und Diatomeenerde; synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Stäube, Pulver oder Granulate vorliegen können.

Als Lösungs- bzw. Dispersionsmittel kommen im wesentlichen in Frage: Aromaten, wie Toluol, Xylole, Benzol und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol sowie deren Aether und Ester; Ketone, wie Aceton, Methyläthylketon, Methylisobutylketon und Cyclohexanon; und stark polare Lösungsmittel, wie Dimethylformamid, N-Methylpyrrolidon und Dimethylsulfoxid, wobei solche Lösungs- bzw. Dispersionsmittel vorzugsweise Flammpunkte von mindestens 30 °C und Siedepunkte von mindestens 50 °C aufweisen, und Wasser. Als Lösungs- bzw. Dispersionsmittel kommen auch sogenannte verflüssigte gasförmige Streckmittel oder Trägerstoffe in Frage, also Produkte, welche bei Raumtemperatur und unter Normaldruck gasförmig sind. Beispiele solcher Produkte sind insbesondere Aerosol-Treibgase, wie Halogenkohlenwasserstoffe, z.B. Dichlordifluormethan. Im Falle der Benutzung von Wasser als Lösungsmittel können z.B. auch organische Lösungsmittel als Hilfslösungsmittel verwendet werden.

Die Tenside (Netzmittel und Emulgatoren) können nicht-ionische Verbindungen darstellen, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen darstellen, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-Dodecylbenzolsulfonat, und Butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen darstellen, wie Alkyldimethylbenzylammoniumchloride, Dialkyldimethylammoniumchloride, Alkyltrimethylammoniumchloride und äthoxylierte quaternäre Ammoniumchloride.

Als Dispergiermittel (ohne Tensidwirkung) kommen im wesentlichen in Frage: Lignin, Natrium- und Ammoniumsalze von Ligninsulfonsäure, Natriumsalze von Maleinsäureanhydrid-Diisobutylen-Copolymeren,

Natrium- und Ammoniumsalze von sulfonierten Polykondensationsprodukten aus Naphthalin und Formaldehyd, und Sulfitablaugen.

Als Dispergiermittel, die sich insbesondere als Verdickungs- bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel, z.B. Epichlorhydrin, Phenylglycidäther und Soyaepoxide; Antioxidantien, z.B. Gallussäureester und Butylhydroxytoluol; UV-Absorber, z.B. substituierte Benzophenone, Diphenylacrylnitrilsäureester und Zimtsäureester; und Deaktivatoren, z.B, Salze der Aethylendiaminotetraessigsäure und Polyglykole.

Die erfindungsgemässen Schädlingsbekämpfungsmittel können neben den Wirkstoffen der Formel I auch andere Wirkstoffe enthalten, z.B. anderweitige Schädlingsbekämpfungsmittel, Schädlingslockstoffe, Fungizide, Bakterizide, Pflanzenwachstumsregulatoren und Düngemittel. Solche Kombinationsmittel eignen sich zur Verstärkung der Aktivität bzw. zur Verbreiterung des Wirkungsspektrums. Gegebenenfalls können dadurch auch Unzulänglichkeiten von bisher bekannten zugesetzten Mitteln ausgeglichen werden.

Es wurde gefunden, dass die erfindungsgemässen Verbindungen I mit Vorteil auch in Kombination mit konventionellen Akariziden, insbesondere mit zur Bekämpfung von Eiern und Larven von Milben geeigneten konventionellen Akariziden, eingesetzt werden. Beispiele derartiger Akarizide sind Chlorbenside, Chlorfenson, Clofentezine, Fenson, Fenothiocarb, Flubenzimine, Tetradifon, Hexythiazox, Benzoximate, Dienochlor, Flufenoxuron und NC-129. Die Anwendung kann gleichzeitig oder getrennt erfolgen. Dabei können die erfindungsgemässen Wirkstoffe den Nachteil bekannter Akarizide mit Wirkungsschwerpunkt gegen die Eier und Larven kompensieren, indem die nach Einsatz dieser bekannten Akarizide überlebenden mobilen Stadien, welche sich rasch zu einer neuen schädlichen Population entwickeln können, ebenfalls abgetötet werden. Da unter Praxisbedingungen oft Eier, verschiedene Larvenstadien sowie Adulte, und zwar männliche und weibliche Formen, gleichzeitig auftreten, kann mit Kombinationspräparaten äusserst praxisbezogene, d.h. raschere, durchschlagendere und länger anhaltende Gesamtwirkung erzielt werden. Die totale Menge der beiden Wirkstoffe in solchen Kombinationspräparaten beträgt aber zweckmässigerweise nicht mehr als die Menge Wirkstoff bei Verwendung einer Verbindung I als alleinigen Wirkstoffes.

Die erfindungsgemässen Schädlingsbekämpfungsmittel enthalten je nach Typ zwischen 0,005 und 95 Gewichtsprozent erfindungsgemässer Verbindung bzw. erfindungsgemässer Verbindungen als Wirkstoff. Sie können in einer Form vorliegen, die sich für die Lagerung und den Transport eignet. In solchen Formen, z.B. emulgierbaren Konzentraten, ist die Wirkstoffkonzentration normalerweise im höheren Bereich der obigen Konzentrationsreihe. Diese Formen können dann mit gleichen oder verschiedenen Formulierungshilfsstoffen bis zu Wirkstoffkonzentrationen verdünnt werden, die sich für den praktischen Gebrauch eignen, und solche Konzentrationen liegen normalerweise im niedrigeren Bereich der obigen Konzentrationsreihe. Emulgierbare Konzentrate enthalten im all gemeinen 5 bis 90 Gewichtsprozent, vorzugsweise 10 bis 80 Gewichtsprozent, der erfindungsgemässen Verbindung(en). Als Anwendungsformen kommen u.a. gebrauchsfertige Lösungen, Emulsionen, Suspensionen, Schäume, Pulver, Pasten, Stäubemittel und Granulate in Frage. Die Wirkstoffkonzentrationen in solchen anwendungsfertigen Zubereitungen können in grossen Bereichen variiert werden. In Spritzbrühen können z.B. Konzentrationen zwischen 0,005 und 0,5 Gewichtsprozent vorliegen. Im Ultra-Low-Volume-Verfahren können Spritzbrühen formuliert werden, in denen die Wirkstoffkonzentration vorzugsweise von 10 bis 20 Gewichsprozent beträgt, während die im Low-Volume-Verfahren und im High-Volume-Verfahren formulierten Spritzbrühen vorzugsweise eine Wirkstoffkonzentration von 0,01 bis 0.5 bzw. 0,005 bis 0,1 Gewichtsprozent aufweisen. Granulate enthalten vorzugsweise von 5 bis 50 Gewichtsprozent der erfindungsgemässen Verbindung(en) als Wirkstoff.

Die erfindungsgemässen Schädlingsbekämpfungsmittel können dadurch hergestellt werden, dass man mindestens eine Verbindung der allgemeinen Formel I bzw. ein Säureadditionssalz einer solchen Verbindung mit Formulierungshilfsstoffen vermischt.

Die Herstellung der Mittel kann in bekannter Weise durchgeführt werden. z.B. durch Vermischen des Wirkstoffes mit festen Trägerstoffen, durch Auflösen oder Suspendieren in geeigneten Lösungs- bzw. Dispersionsmitteln, eventuell unter Verwendung von Tensiden als Netzmitteln oder Emulgatoren oder von Dispergiermitteln, durch Verdünnen bereits vorbereiteter emulgierbarer Konzentrate mit Lösungs- bzw. Dispersionsmitteln, usw.

Im Falle von pulverförmigen Mitteln kann der Wirkstoff mit einem festen Trägerstoff vermischt werden, z.B. durch Zusammenmahlen; oder man kann den festen Trägerstoff mit einer Lösung oder Suspension des Wirkstoffes imprägnieren und dann das Lösungs- oder Suspensionsmittel durch Abdunsten, Erhitzen oder durch Absaugen unter vermindertem Druck entfernen. Durch Zusatz von Tensiden bzw. Dispergiermitteln kann man solche pulverförmige Mittel mit Wasser leicht benetzbar machen, so dass sie in wässrige Suspensionen, die sich z.B. als Spritzmittel eignen, übergeführt werden können.

Die Verbindungen der Formel I bzw. deren Säureadditionssalze können auch mit einem Tensid und einem festen Trägerstoff zur Bildung eines netzbaren Pulvers vermischt werden, welches in Wasser dispergierbar ist, oder sie können mit einem festen granulierten Trägerstoff zur Bildung eines Granulates vermischt werden.

Wenn gewünscht, kann die Verbindung der Formel I oder ein Säureadditionssalz davon in einem mit Wasser nicht mischbaren Lösungsmittel, wie beispielsweise einem alicyclischen Keton, gelöst werden, das zweckmässigerweise gelösten Emulgator enthält, so dass die Lösung bei Zugabe zu Wasser selbstemülgierend wirkt. Andernfalls kann der Wirkstoff mit einem Emulgator vermischt und das Gemisch dann mit Wasser auf die gewünschte Konzentration verdünnt werden. Zudem kann der Wirkstoff in einem Lösungsmittel gelöst und danach mit einem Emulgator gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Das erfindungsgemässe Verfahren zur Bekämpfung von Schädlingen ist dadurch gekennzeichnet, dass man das zu schützende Gut oder die Schädlinge selbst mit einer wirksamen Menge einer erfindungsgemässen Verbindung bzw. eines erfindungsgemässen Schädlingsbekämpfungsmittels behandelt. Dieses Anwendungsverfahren kann durch Boden- oder Blattapplikation, bzw. durch Applikation auf die zu schützenden Tiere, Vorräte oder Materialien, je nach Art der zu bekämpfenden Schädlinge, durchgeführt werden. Die Bekämpfung wird beispielsweise durch Kontakt oder durch Einnahme mit der Nahrung erzielt.

Die Anwendung kann in konventioneller Weise geschehen, z.B. durch Verspritzen, Versprühen, Vernebeln, Verstäuben, Verstreuen, Eindrillen, Verräuchern, Giessen, Beizen oder Inkrustieren. Pulverförmige Präparate können z.B. als Stäubemittel mit Hilfe der üblichen Verstäubegeräte auf die Schädlinge bzw. auf das zu schützende Gut, z.B. Pflanzen oder Tiere, aufgebracht werden. Wässrige Suspensionen sind z.B. als Spritzmittel anwendbar.

Bei der Anwendung im Pflanzenschutz genügt in der Regel eine Dosierung von ca. 100-500 g Wirkstoff [Verbindungen(en) der Formel I]/ha, z.B. wie dies bei der Applikation von 2000 l einer Spritzbrühe, die 0,005-0,025 Gewichtsprozent Wirkstoff enthält, auf 1 ha bepflanzten Erdbodens der Fall ist.

Die nachfolgenden Beispiele dienen zur näheren Erläuterung der Erfindung.

I. Herstellung der Wirkstoffe der Formel I:

Beispiel 1

Ein Gemisch bestehend aus 11,0 g 5,7-Dihydro-6H-dibenz[c,e]azepin-6-carbonitril, 11.7 g Aceton-(2-hydroxyäthyl)oxim und 0,33 g Kaliumcyanid wird 3 Tage auf 85 °C erwärmt. Das auf ca. 40 °C abgekühlte Reaktionsgemisch wird mit 100 ml Wasser versetzt und zweimal mit Essigester extrahiert. Die Extrakte werden dreimal mit Wasser und je einmal mit halbgesättigter und gesättigter Kochsalzlösung gewaschen, über wasserfreiem Natriumsulfat getrocknet und unter vermindertem Druck eingedampft. Der Rückstand wird an Kieselgel mit Aceton als Laufmittel chromatographiert. Man erhält den 5,7-Dihydro-6H-dibenz[c,e]-azepin -6-carboximidsäure-{2-[(isopro pylidenamino)oxy] -äthyl}ester als Oel, Massenspektrum m/e: $M^+$ 337 (13), 281 (11), 237 (72), 194 (84), 179 (79), 165 (78), 100 (85), 56 (100).

In analoger Weise erhält man ausgehend von

- 5,7-Dihydro-6H-dibenz[c,e]azepin-6-carbonitril und Benzylalkohol den 5,7-Dihydro-6H-dibenz[c,e]azepin-6-carboximidsäure-benzylester als Oel, Massenspektrum m/e: $M^+$ 328 (4), 237 (44), 194 (100), 179 (21), 165 (30), 91 (62);
- 5,7-Dihydro-6H-dibenz[c,e]azepin-6-carbonitril und Acetonoxim das Aceton-O-[(5,7-dihydro-6H-dibenz[c,e]-azepin-6-yl)carboximidoyl]oxim als Sirup, Massenspektrum m/e: $M^+$ 294 (0,5), 237 (35), 220 (38), 194 (100), 179 (63), 165 (78);
- 5,7-Dihydro-6H-dibenz[c,e]azepin-6-carbonitril und 2-Hydroxy-äthylcarbaminsäure-äthylester den 5,7-Dihydro-6H-dibenz[c,e]azepin -6-carboximidsäure-(2-äthoxycarbonylamino-äthyl)ester als harzartiges Produkt, Massenspektrum m/e: $M^+$ 353 (2), 308 (4), 237 (32), 194 (78), 116 (100), 88 (61);
- 5,7-Dihydro-6H-dibenz[c,e]azepin-6-carbonitril und m-Phenoxybenzylalkohol den 5,7-Dihydro-6H-dibenz-[c,e]azepin-6-carboximidsaure-(m-phenoxybenzyl)ester als Sirup, Massenspektrum m/e: $M^+$ 420 (14), 327 (13), 237 (35), 194 (100);
- 5,7-Dihydro-6H-dibenz[c,e]azepin-6-carbonitril und p-Chlorbenzylalkohol den 5,7-Dihydro-6H-dibenz[c,e]-azepin-6-carboximidsäure-(p-chlorbenzyl)ester, Smp. 103-105 °C;

- 5,7-Dihydro-6H-dibenz[c,e]azepin-6-carbonitril und 2-Butanon-(2-hydroxyäthyl)oxim den 5,7-Dihydro-6H-dibenz[c,e]azepin-6-carboximidsäure {2-[(1-methylpropyliden amino)oxy]-äthyl}ester als Sirup, Massenspektrum m/e: $M^+$ 351 (23), 281 (5), 237 (57), 194 (100), 114 (100), 70 (12);
- 5,7-Dihydro-6H-dibenz[c,e]azepin-6-carbonitril und Aceton-(2-hydroxypropyl)oxim den 5,7-Dihydro-6H-dibenz[c,e]azepin-6-carboximidsäure-{2-[(isopropylidenamino)oxy]-1-methyläthyl}ester als Sirup, Massenspektrum m/e: $M^+$ 351 (12), 295 (4), 237 (81), 194 (100), 114 (35), 56 (78);
- 5,7-Dihydro-6H-dibenz[c,e]azepin-6-carbonitril und 2-Hydroxy-äthylcarbaminsäure-methylester den 5,7-Dihydro-6H-dibenz[c,e]azepin-6-carboximidsäure-(2-methoxycarbonylaminoäthyl)ester als Harz, Massenspektrum m/e: $M^+$ 339 (5), 308 (2), 237 (22), 194 (56), 102 (100);
- 5,7-Dihydro-6H-dibenz[c,e]azepin-6-carbonitril und 2-Hydroxy-äthyl-N-methylcarbaminsäure-äthylester den 5,7-Dihydro-6H-dibenz[c,e]azepin-6-carboximinsäure-[2-(N-methyl-äthoxycarbonylamino)-äthyl]ester als Sirup, Massenspektrum m/e: $M^+$ 367 (2), 322 (2), 237 (6), 194 (26), 130 (100), 102 (59);
- 5,7-Dihydro-6H-dibenz[c,e]azepin-6-carbonitril und 2-Hydroxymethyl-tetrahydrofuran den 5,7-Dihydro 6H-dibenz[c,e]azepin-6-carboximidsäure-(2-tetrahydrofurylmethyl)ester als Sirup, Massenspektrum m/e: $M^+$ 322 (14), 237 (66), 194 (100), 85 (36), 71 (12);
- 5,7-Dihydro-6H-dibenz[c,e]azepin-6-carbonitril und 2-Hydroxymethyl-tetrahydropyran den 5,7-Dihydro-6H-dibenz[c,e]azepin-6-carboximidsäure-(2-tetrahydro-2H-pyranylmethyl)ester als Sirup, Massenspektrum m/e: $M^+$ 336 (30), 237 (78), 194 (100), 99 (60);
- 5,7-Dihydro-6H-dibenz[c,e]azepin-6-carbonitril und 3-Phenyl-propan-1-ol den 5,7-Dihydro-6H-dibenz[c,e]-azepin-6-carboximidsäure-(3-phenylpropyl)ester als Harz, Massen spektrum m/e: $M^+$ 356 (15), 237 (68), 194 (100), 117 (8), 91 (30);
- 5,7-Dihydro-6H-dibenz[c,e]azepin-6-carbonitril und 2-Phenyläthanol den 5,7-Dihydro-6H-dibenz[c,e]azepin-6-carboximidsäure-(2-phenyläthyl)ester als Harz, Massenspektrum m/e: $M^+$ 342 (18), 237 (71), 194 (100), 105 (18);
- 5,7-Dihydro-6H-dibenz[c,e]azepin-6-carbonitril und α-Methylbenzylalkohol den 5,7-Dihydro-6H-dibenz[c,e]-azepin-6-carboximidsäure-(α-methylbenzyl)ester als Harz, Massenspektrum m/e: $M^+$ 342 (5), 237 (60), 194 (100), 105 (63);
- 5,7-Dihydro-6H-dibenz[c,e]azepin-6-carbonitril und 2-Hydroxymethylnaphthalin den 5,7-Dihydro-6H-dibenz-[c,e]azepin-6-carboximidsäure-(2-naphthylmethyl)ester als Harz, Massenspektrum m/e: $M^+$ 378 (19), 237 (15), 194 (90), 141 (100), 115 (53);
- 5,7-Dihydro-6H-dibenz[c,e]azepin-6-carbonitril und 2-Phenoxyäthanol den 5,7-Dihydro-6H-dibenz[c,e]-azepin-6-carboximidsäure-(2-phenoxyäthyl)ester als Harz, Massenspektrum m/e: $M^+$ 358 (20), 265 (7), 237 (65), 194 (100);
- 5,7-Dihydro-6H-dibenz[c,e]azepin-6-carbonitril und N-(2-Hydroxyäthyl)pyrrolidin den 5,7-Dihydro-6H-dibenz[c,e]azepin-6-carboximidsäure-(2-pyrrolidino-äthyl)ester als Harz, Massenspektrum m/e: $M^+$ 335 (1), 238 (6), 194 (12), 179 (22), 165 (22), 97 (100), 84 (39), 69 (56);
- 5,7-Dihydro-6H-dibenz[c,e]azepin-6-carbonitril und 1-(2-Hydroxyäthyl)-pyrrolidon den 5,7-Dihydro-6H-dibenz[c,e]azepin-6-carboximidsäure-[2-(2-pyrrolidinon)-äthyl]ester als Harz, Massenspektrum m/e: $M^+$ 349 (2), 238 (20), 194 (41), 112 (100);
- 5,7-Dihydro-6H-dibenz[c,e]azepin-6-carbonitril und 4-(2-Hydroxyäthyl)-morpholin den 5,7-Dihydro-6H-dibenz[c,e]azepin-6-carboximidsäure-(2-morpholino-äthyl)ester als Harz, Massenspektrum m/e: $M^+$ 351 (1), 238 (10), 113 (100);
- 5,7-Dihydro-6H-dibenz[c,e]azepin-6-carbonitril und 2-(2-Methoxyäthoxy)-äthanol den 5,7-Dihydro-6H-dibenz[c,e]azepin-6-carboximidsäure-[2-(2-methoxyäthoxy)-äthyl]ester als Harz, Massenspektrum m/e: $M^+$ 340 (20), 237 (80), 194 (100), 59 (45).

## Beispiel 2

20 ml 2-Methoxyäthanol werden bei 60 °C portionenweise mit 0,23 g Natrium versetzt. Nach 30 Minuten werden 2,2 g 5,7-Dihydro-6H-dibenz[c,e]azepin-6-carbonitril zugegeben, und das Gemisch wird während 1 Stunde bei 80 °C gerührt. Anschliessend wird das 2-Methoxyäthanol bei reduziertem Druck abdestilliert, der Rückstand auf Eiswasser gegossen und das resultierende Gemisch zweimal mit Methylenchlorid extrahiert. Man wäscht die vereinigten Extrakte zweimal mit Wasser, trocknet sie über wasserfreiem Natriumsulfat und dampft sie ein. Der Rückstand wird an Kieselgel mit Essigester und steigenden Mengen Aethanol als Laufmittel chromatographiert. Man erhält den 5,7-Dihydro-6H-dibenz[c,e]azepin -6-carboximidsäure-(2-methoxyäthyl)ester als Sirup, Massenspektrum m/e: $M^+$ 296 (6), 237 (45), 194 (100), 178 (74), 165 (49).

In analoger Weise erhält man ausgehend von

- 5,7-Dihydro-6H-dibenz[c,e]azepin-6-carbonitril und Hydroxymethylcyclopropan den 5,7-Dihydro-6H-dibenz-[c,e]azepin-6-carboximidsäure-cyclopropylmethylester, Smp. 80-83 °C, Massenspektrum m/e: $M^+$ 292 (2), 237 (80), 194 (100);
- 5,7-Dihydro-6H-dibenz[c,e]azepin-6-carbonitril und Thiophenol den 5,7-Dihydro-6H-dibenz[c,e]azepin -6-thio carboximidsäure-phenylester als Sirup, Massenspektrum m/e: $M^+$ 330 (3), 220 (70), 179 (100), 165 (85), 110 (74);

## Beispiel 3

Man löst 1,0 g 5,7-Dihydro-6H-dibenz[c,e]azepin-6-carboximidsäure-benzylester in 1 ml Methanol, kühlt die Lösung auf 0 °C und versetzt sie mit 0,6 ml einer 5N alkoholischen Salzsäure-Lösung. Nach zehnminütigem Rühren bei 0-5 °C werden 10 ml n-Hexan zugegeben, und das ausgefallene Produkt wird abgenutscht. Man erhält das 5,7-Dihydro-6H-dibenz[c,e]azepin -6-carboximidsäure-benzylester-hydrochlorid, Smp. 90-92 °C.

## Beispiel 4

9,32 g 5,7-Dihydro-6H-dibenz[c,e]azepin -6-carboximidsäure-äthylester werden mit 3,54 g Triäthylamin in 100 ml tert.Butylmethyläther vorgelegt. Unter Eisbadkühlung werden 4,92 g Benzoylchlorid innert ca. 30 Minuten zugetropft. Dann lässt man 20 Minuten bei 0-5 °C nachrühren. Das Reaktionsgemisch wird auf Wasser gegossen und zweimal mit Essigester extrahiert. Die Extrakte werden mit Wasser und gesättigter Kochsalzlösung gewaschen, über wasserfreiem Natriumsulfat getrocknet und eingedampft. Das Rohprodukt wird in Essigester warm gelöst und über eine ca. 3 cm hohe Schicht von Kieselgel abgenutscht. Die nach Eindampfen des Filtrates erhaltenen Kristalle werden aus Aethanol umkristallisiert. Man erhält den N-Benzoyl-5,7-dihydro-6H-dibenz[c,e]azepin -6-carboximidsäure-äthylester, Smp. 116-117 °C.

In analoger Weise erhält man ausgehend von

- 5,7-Dihydro-6H-dibenz[c,e]azepin -6-carboximidsäure-äthylester und p-Trifluormethylbenzoylchlorid den N-(p-Trifluormethylbenzoyl)-5,7-dihydro-6H-dibenz[c,e]azepin-6-carboximidsäure-äthylester, Smp. 112-113 °C;
- 5,7-Dihydro-6H-dibenz[c,e]azepin -6-carboximidsäure-äthylester und p-Fluorbenzoylchlorid den N-(p-Fluorbenzoyl)-5,7-dihydro-6H-dibenz[c,e]azepin -6-carboximidsäure-äthylester, Smp. 109-110 °C;
- 5,7-Dihydro-6H-dibenz[c,e]azepin -6-carboximidsäure-äthylester und p-Chlorbenzoylchlorid den N-(p-Chlorbenzoyl)-5,7-dihydro-6H-dibenz[c,e]azepin -6-carboximidsäure-äthylester, Smp. 106-107 °C;
- 5,7-Dihydro-6H-dibenz[c,e]azepin -6-carboximidsäure-äthylester und p-Toluoylchlorid den N-(p-Toluoyl)-5,7-dihydro-6H-dibenz[c,e]azepin -6-carboximidsäure-äthylester als Harz, Massenspektrum m/e: $M^+$ 384 (9), 355 (15), 194 (100);
- 5,7-Dihydro-6H-dibenz[c,e]azepin -6-carboximidsäure-äthylester und p-Anisoylchlorid den N-(p-Anisoyl)-5,7-dihydro-6H-dibenz[c,e]azepin -6-carboximidsäure-äthylester, Smp. 71-78 °C;
- 5,7-Dihyro-6H-dibenz[c,e]azepin -6-carboximidsäure-(2-äthoxycarbonylamino-äthyl)ester und Benzoylchlorid den N-Benzoyl-5,7-dihydro-6H-dibenz[c,e]azepin -6-carboximidsäure-(2-äthoxycarbonylamino-äthyl)ester als Harz, Massenspektrum m/e: $M^+$ 457 (6), 341 (18), 194 (100), 116 (75), 105 (75);
- 5,7-Dihydro-6H-dibenz[c,e]azepin -6-thiocarboximidsäure-dodecylester und Benzoylchlorid den N-Benzoyl-5,7-dihydro-6H-dibenz[c,e]azepin -6-thiocarboximidsäure-dodecylester als Harz, Massenspektrum m/e: $M^+$ 526 (23), 357 (18), 194 (94), 105 (100);
- 5,7-Dihydro-6H-dibenz[c,e]azepin -6-carboximidsäure-{2-[(isopropylidenamino)oxy] -äthyl}ester und Benzoylchlorid den N-Benzoyl-5,7-dihydro-6H-dibenz[c,e]azepin-6-carboximidsäure-{2-[(isopropylidenamino)-oxy] -äthyl}ester als Harz, Massenspektrum m/e: $M^+$ 441 (11), 341 (39), 194 (100), 105 (60), 100 (60);
- 5,7-Dihydro-6H-dibenz[c,e]azepin -6-carboximidsäure-äthylester und 2,6-Difluorbenzoylchlorid den N-(2,6-Difluorbenzoyl)-5,7-dihydro-6H-dibenz[c,e]azepin -6-carboximidsäure-äthylester als Harz, Massenspektrum m/e: $M^+$ 406 (5), 377 (21), 194 (100), 141 (42);
- 5,7-Dihydro-6H-dibenz[c,e]azepin -6-carboximidsäure-methylester und Benzoylchlorid den N-Benzoyl-5,7-dihydro-6H-dibenz[c,e]azepin -6-carboximidsäure-methylester, Smp. 130-131 °C;
- 5,7-Dihydro-6H-dibenz[c,e]azepin -6-carboximidsäure-propylester und Benzoylchlorid den N-Benzoyl-5,7-dihydro-6H-dibenz[c,e]azepin -6-carboximidsäure-propylester als Harz, Massenspektrum m/e: $M^{+}$384 (7), 341 (13), 194 (100), 179 (23), 105 (59);
- 5,7-Dihydro-6H-dibenz[c,e]azepin -6-carboximidsäure-äthylester und 3,5-Bis-(trifluormethyl)benzoylchlorid den N-[3,5-Bis-(trifluormethyl)-benzoyl]-5,7-dihydro-6H-dibenz[c,e]azepin-6-carboximidsäure-äthylester, Smp. 129-130 °C;
- 5,7-Dihydro-6H-dibenz[c,e]azepin -6-carboximidsäure-äthylester und 3,4-Dichlorbenzoylchlorid den N-(3,4-Dichlorbenzoyl)-5,7-dihydro-6H-dibenz[c,e]azepin -6-carboximidsäure-äthylester, Smp. 138 °C;
- 5,7-Dihydro-6H-dibenz[c,e]azepin -6-carboximidsäure-äthylester und 2-Furoylchlorid den N-(2-Furoyl)-5,7-dihydro-6H-dibenz[c,e]azepin -6-carboximidsäure-äthylester, Smp. 87-92 °C;
- 5,7-Dihydro-6H-dibenz[c,e]azepin -6-carboximidsäure-äthylester und Cyclohexancarbonsäurechlorid den N-Cyclohexylcarbonyl-5,7-dihydro-6H-dibenz[c,e]azepin -6-carboximidsäure-äthylester als Harz, Massenspektrum m/e: $M^{+}$ 376 (13), 347 (16), 293 (100), 194 (60);
- 5,7-Dihydro-6H-dibenz[c,e]azepin -6-carboximidsäure-äthylester und Cyclopropancarbonsäurechlorid den N-Cyclopropylcarbonyl-5,7-dihydro-6H-dibenz[c,e]azepin -6-carboximidsäure-äthylester, Smp. 97-99 °C;
- 5,7-Dihydro-6H-dibenz[c,e]azepin -6-carboximidsäure-äthylester und Biphenyl-4-carbonsäurechlorid den N-(4-Biphenylylcarbonyl)-5,7-dihydro-6H-dibenz[c,e]azepin-6-carboximidsäure-äthylester, Smp. 52-58 °C;
- 5,7-Dihydro-6H-dibenz[c,e]azepin -6-carboximidsäure-äthylester und 2,6-Dichlorpyridin-4-carbonsäurechlorid den N-(2,6-Dichlorisonicotinoyl)-5,7-dihydro-6H-dibenz[c,e]azepin-6-carboximidsäure-äthylester, Smp. 151-152 °C;
- 5,7-Dihydro-6H-dibenz[c,e]azepin -6-carboximidsäure-äthylester und Acetylchlorid den N-Acetyl-5,7-dihydro-6H-dibenz[c,e]azepin -6-carboximisäure-äthylester als Sirup, Massenspektrum m/e: $M^{+}$ 308 (2), 279 (25), 194 (100);
- 5,7-Dihydro-6H-dibenz[c,e]azepin -6-carboximidsäure-äthylester und Pivalinsäurechlorid den N-Pivaloyl-5,7-dihydro-6H-dibenz[c,e]azepin -6-carboximidsäure-äthylester als Sirup, Massenspektrum m/e: $M^{+}$ 350 (2), 321 (5), 293 (100), 265 (18), 194 (29), 167 (92);
- 5,7-Dihydro-6H-dibenz[c,e]azepin -6-carboximidsäure-äthylester und 2-Trifluormethylbenzoylchlorid den N-(o-Trifluormethylbenzoyl)-5,7-dihydro-6H-dibenz[c,e]azepin-6-carboximidsäure-äthylester, Smp. 122-125 °C;
- 5,7-Dihydro-6H-dibenz[c,e]azepin -6-carboximidsäure-äthylester und Chloracetylchlorid den N-Chloracetyl-5,7-dihydro-6H-dibenz[c,e]azepin -6-carboximidsäure-äthylester, Smp. 87-89 °C;
- 5,7-Dihydro-6H-dibenz[c,e]azepin-6-carboximidsäure-äthylester und 3,4-Dimethoxybenzoylchlorid den N-(3,4-Dimethoxybenzoyl)-5,7-dihydro-6H-dibenz[c,e]azepin-6-carboximidsäure-äthylester als Feststoff, Massenspektrum m/e: $M^{+}$ 430 (12), 401 (5), 194 (100), 165 (42);
- 5,7-Dihydro-6H-dibenz[c,e]azepin-6-carboximidsäure-äthylester und Isonicotinsäurechlorid den N-(Isonicotinoyl)-5,7-dihydro-6H-dibenz[c,e]azepin-6-carboximidsäure-äthylester, Smp. 120-122 °C;
- 5,7-Dihydro-6H-dibenz[c,e]azepin-6-carboximidsäure-äthylester und Nicotinsäurechlorid den N-(Nicotinoyl)-5,7-dihydro-6H-dibenz[c,e]azepin-6-carboximidsäure-äthylester als Harz, Massenspektrum m/e: $M^{+}$ 371 (5), 342 (26), 194 (100), 106 (38);
- 5,7-Dihydro-6H-dibenz[c,e]azepin-6-carboximidsäure-äthylester und Acrylsäurechlorid den N-(Acryloyl)-5,7-dihydro-6H-dibenz[c,e]azepin-carboximidsäure-äthylester als Sirup, Massenspektrum m/e: $M^{+}$ 320 (11), 291 (19), 194 (100), 55 (15);
- 5,7-Dihydro-6H-dibenz[c,e]azepin-6-carboximidsäure-äthylester und 2-Methylacrylsäurechlorid den N-(2-Methylacryloyl)-5,7-dihydro-6H-dibenz[c,e]azepin-6-carboximidsäure-äthylester als Sirup, Massenspektrum m/e: $M^{+}$ 334 (20), 305 (16), 293 (7), 194 (100), 167 (29), 69 (21), 41 (30);
- 5,7-Dihydro-6H-dibenz[c,e]azepin-6-carboximidsäure-äthylester und p-Cyanobenzoylchlorid den N-(p-Cyanobenzoyl)-5,7-dihydro-6H-dibenz[c,e]azepin-6-carboximidsäure-äthylester als Feststoff, Massenspektrum m/e: $M^{+}$ 395 (5), 366 (24), 194 (100), 165 (18), 130 (20), 102 (14);
- 5,7-Dihydro-6H-dibenz[c,e]azepin-6-carboximidsäure-äthylester und 2,4-Dichlorbenzoylchlorid den N-(2,4-Dichlorbenzoyl)-5,7-dihydro-6H-dibenz[c,e]azepin-6-carboximidsäure-äthylester, Smp. 158-159 °C;
- 5,7-Dihydro-6H-dibenz[c,e]azepin-6-carboximidsäure-äthylester und Sorbinsäurechlorid den N-[(all-E)-2,4-hexadienoyl)-5,7-dihydro-6H-dibenz[c,e]azepin-6-carboximidsäure-äthylester als Sirup, Massenspektrum m/e: $M^{+}$ 360 (13), 331 (5), 305 (10), 194 (100), 95 (25);
- 5,7-Dihydro-6H-dibenz[c,e]azepin-6-carboximidsäure-äthylester und Zimtsäurechlorid den N-[(E)-Anisamoyl]-5,7-dihydro-6H-dibenz[c,e]azepin-6-carboximidsäure-äthylester als Harz, Massenspektrum m/e: $M^{+}$ 396 (15), 367 (4), 194 (100), 131 (21);
- 5,7-Dihydro-6H-dibenz[c,e]azepin-6-carboximidsäure-äthylester und Phenylessigsäurechlorid den N-(Phenylacetyl)-5,7-dihydro-6H-dibenz[c,e]azepin-6-carboximidsäure-äthylester als Sirup, Massenspektrum m/e: $M^{+}$ 384 (2), 355 (1), 293 (100), 265 (15), 194 (11), 179 (26), 167 (67), 91 (20);
- 5,7-Dihydro-6H-dibenz[c,e]azepin-6-carboximidsäure-äthylester und 2-Thienylcarbonsäurechlorid den N-(2-Thienylcarbonyl)-5,7-dihydro-6H-dibenz[c,e]azepin-6-carboximidsäure-äthylester als Feststoff, Massenspektrum m/e: $M^+$ 376 (10), 347 (13), 194 (100), 111 (48);

- 5,7-Dihydro-6H-dibenz[c,e]azepin-6-carboximidsäure-äthylester und 1-Naphthoesäurechlorid den N-(1-Naphthylcarbonyl)-5,7-dihydro-6H-dibenz[c,e]azepin-6-carboximidsäure-äthylester als Schaum, Massenspektrum m/e: $M^+$ 420 (8), 391 (6), 194 (100), 155 (32), 127 (28);

- 5,7-Dihydro-6H-dibenz[c,e]azepin-6-carboximidsäure-äthylester und 2-Naphthoesäurechlorid den N-(2-Naphthylcarbonyl)-5,7-dihydro-6H-dibenz[c,e]azepin-6-carboximidsäure-äthylester als Schaum, Massenspektrum m/e: $M^+$ 420 (7), 391 (8), 194 (100), 155 (28), 127 (28);

- 5,7-Dihydro-6H-dibenz[c,e]azepin-6-carboximidsäure-äthylester und p-tert.Butylbenzoylchlorid den N-(p-tert.Butylbenzoyl)-5,7-dihydro-6H-dibenz[c,e]azepin-6-carboximidsäure-äthylester als Schaum, Massenspektrum m/e: $M^+$ 426 (8), 398 (6), 194 (100), 161 (36);

- 5,7-Dihydro-6H-dibenz[c,e]azepin-6-carboximidsäure-äthylester und p-Brombenzoylchlorid den N-(p-Brombenzoyl)-5,7-dihydro-6H-dibenz[c,e]azepin-6-carboximidsäure-äthylester, Smp. 116-117° C,

- 5,7-Dihydro-6H-dibenz[c,e]azepin-6-carboximidsäure-propylester und p-Fluorbenzoylchlorid den N-(p-Fluorbenzoyl)-5,7-dihydro-6H-dibenz[c,e]azepin-6-carboximidsäure-propylester als Feststoff, Massenspektrum m/e: $M^+$ 402 (5), 359 (14), 194 (100), 123 (51);

- 5,7-Dihydro-6H-dibenz[c,e]azepin-6-carboximidsäure-isopropylester und p-Fluorbenzoylchlorid den N-(p-Fluorbenzoyl)-5,7-dihydro-6H-dibenz[c,e]azepin-6-carboximidsäure-isopropylester, Smp. 90-93° C;

- 5,7-Dihydro-6H-dibenz[c,e]azepin-6-carboximidsäure-methylester und p-Fluorbenzoylchlorid den N-(p-Fluorbenzoyl)-5,7-dihydro-6H-dibenz[c,e]azepin-6-carboximidsäure-methylester, Smp. 136-137,5° C;

- 5,7-Dihydro-6H-dibenz[c,e]azepin-6-carboximidsäure-äthylester und o-Fluorbenzoylchlorid den N-(o-Fluorbenzoyl)-5,7-dihydro-6H-dibenz[c,e]azepin-6-carboximidsäure-äthylester als Sirup, Massenspektrum m/e: $M^+$ 388 (6), 359 (15), 194 (100), 123 (43);

- 5,7-Dihydro-6H-dibenz[c,e]azepin-6-carboximidsäure-äthylester und m-Fluorbenzoylchlorid den N-(m-Fluorbenzoyl)-5,7-dihydro-6H-dibenz[c,e]azepin-6-carboximidsäure-äthylester als Sirup, Massenspektrum m/e: $M^+$ 388 (3), 359 (13), 194 (100), 123 (46), 95 (36);

- 5,7-Dihydro-6H-dibenz[c,e]azepin-6-carboximidsäure-äthylester und 2,4-Difluorbenzoylchlorid den N-(2,4-Difluorbenzoyl)-5,7-dihydro-6H-dibenz[c,e]azepin-6-carboximidsäure-äthylester als Sirup, Massenspektrum m/e: $M^+$ 406 (8), 377 (22), 194 (100), 141 (74);

- 5,7-Dihydro-6H-dibenz[c,e]azepin-6-carboximidsäure-äthylester und 2,5-Difluorbenzoylchlorid den N-(2,5-Difluorbenzoyl)-5,7-dihydro-6H-dibenz[c,e]azepin-6-carboximidsäure-äthylester als Sirup, Massenspektrum m/e: $M^+$ 406 (4), 377 (18), 194 (100), 141 (49);

- 5,7-Dihydro-6H-dibenz[c,e]azepin-6-carboximidsäure-äthylester und 3,4-Difluorbenzoylchlorid den N-(3,4-Difluorbenzoyl)-5,7-dihydro-6H-dibenz[c,e]azepin-6-carboximidsäure-äthylester als Harz, Massenspektrum m/e: $M^+$ 406 (3), 377 (17), 194 (100), 141 (38);

- 5,7-Dihydro-6H-dibenz[c,e]azepin-6-carboximidsäure-äthylester und 3,5-Difluorbenzoylchlorid den N-(3,5-Difluorbenzoyl)-5,7-dihydro-6H-dibenz[c,e]azepin-6-carboximidsäure-äthylester als Feststoff, Massenspektrum m/e: $M^+$ 406 (4), 377 (20), 194 (100), 141 (35);

- 5,7-Dihydro-6H-dibenz[c,e]azepin-6-carboximidsäure-äthylester und p-(Trifluormethoxy)-benzoylchlorid den N-[p-(Trifluormethoxy)-benzoyl]-5,7-dihydro-6H-dibenz[c,e]azepin-6- carboximidsäure-äthylester als Sirup, Massenspektrum m/e: $M^+$ 454 (1), 425 (11), 194 (100), 189 (40);

- 5,7-Dihydro-6H-dibenz[c,e]azepin-6-carboximidsäure-äthylester und o-Tolylsäurechlorid den N-(o-Toluoyl)-5,7-dihydro-6H-dibenz[c,e]azepin-6-carboximidsäure-äthylester als Sirup, Massenspektrum m/e: $M^+$ 384 (5), 355 (10), 194 (100), 119 (31);

- 5,7-Dihydro-6H-dibenz[c,e]azepin-6-carboximidsäure-äthylester und N-Morpholincarbonylchlorid den N-(Morpholincarbonyl)-5,7-dihydro-6H-dibenz[c,e]azepin-6-carboximidsäure-äthylester als Harz, Massenspektrum m/e: $M^+$ 379 (12), 350 (22), 293 (77), 194 (100), 167 (98), 114 (5);

- Aceton-O-[(5,7-dihydro-6H-dibenz[c,e]azepin-6-yl)carboximidoyl]oxim und Benzoylchlorid das N-{(5,7-Dihydro-6H-dibenz[c,e]azepin-6-yl)[(isopropylidenamino)oxy]methylen}benzamid als Feststoff, Massenspektrum m/e; 341 (13, M-56), 194 (100), 105 (80), 56 (44);

- 5,7-Dihydro-6H-dibenz[c,e]azepin-6-carboximidsäure-(2-tetrahydrofurylmethyl)ester und Benzoylchlorid den N-(Benzoyl)-5,7-dihydro-6H-dibenz[c,e]azepin-6-carboximidsäure-(tetrahydro-2-furfuryl)ester, Smp. 158-159° C;

- 5,7-Dihydro-6H-dibenz[c,e]azepin-6-carboximidsäure-(2-tetrahydro-2H-pyranylmethyl)ester und Benzoylchlorid den N-(Benzoyl)-5,7-dihydro-6H-dibenz[c,e]azepin-6-carboximidsäure-(tetrahydro-2H-pyran-2-yl)-ester, Smp. 114,5-117° C;

- 5,7-Dihydro-6H-dibenz[c,e]azepin-6-carboximidsäure (3-pentinyl)ester und Benzoylchlorid den N-(Benzoyl)-5,7-dihydro-6H-dibenz[c,e]azepin-6-carboximidsäure-(3-pentinyl)ester als Sirup, Massenspektrum

m/e: $M^+$ 408 (3), 407 (4), 341 (8), 194 (100), 105 (57);
- 5,7-Dihydro-6H-dibenz[c,e]azepin-6-carboximidsäure-äthylester und 3,4-Methylendioxybenzoylchlorid den N-(3,4-Methylendioxybenzoyl)-5,7-dihydro-6H-dibenz[c,e]azepin-6-carboximidsäure-äthylester als Sirup, Massenspektrum m/e: $M^+$ 414 (5), 385 (7), 194 (100), 149 (33);
- 5,7-Dihydro-6H-dibenz[c,e]azepin-6-carboximidsäure-äthylester und m-Tolylsäurechlorid den N-(m-Toluoyl)-5,7-dihydro-6H-dibenz[c,e]azepin-6-carboximidsäure-äthylester, Smp. 102-105° C;
- 5,7-Dihydro-6H-dibenz[c,e]azepin-6-carboximidsäure-äthylester und p-Phenoxybenzoylchlorid den N-(p-Phenoxybenzoyl)-5,7-dihydro-6H-dibenz[c,e]azepin-6-carboximidsäue-äthylester als Schaum, Massenspektrum m/e: $M^+$ 462 (8), 433 (5), 197 (30), 194 (100);
- 5,7-Dihydro-6H-dibenz[c,e]azepin-6-carboximidsäure-äthylester und Methoxyessigsäurechlorid den N-(Methoxyacetyl)-5,7-dihydro-6H-dibenz[c,e]azepin-6-carboximidsäure-äthylester als Sirup, Massenspektrum m/e: 339 (1, M+H), 309 (1), 293 (98), 194 (20), 167 (100);
- 5,7-Dihydro-6H-dibenz[c,e]azepin-6-carboximidsäure-äthylester und Methansulfochlorid den N-(Methylsulfonyl)-5,7-dihydro-6H-dibenz[c,e]azepin-6-carboximidsäure-äthylester, Smp. 136,5-138° C;
- 5,7-Dihydro-6H-dibenz[c,e]azepin-6-carboximidsäure-äthylester und Benzolsulfochlorid den N-(Phenylsulfonyl)-5,7-dihydro-6H-dibenz[c,e]azepin 6-carboximidsäure-äthylester, Smp. 145,5-149,5° C.

## Beispiel 5

2,66 g 5,7-Dihydro 6H-dibenz[c,e]azepin 6-carboximidsäure-äthylester werden mit 1 g Triäthylamin in 15 ml Methylenchlorid vorgelegt. Unter Eisbadkühlung werden 0,95 g Chlorameisensäure-methylester zugetropft. Dann lässt man 2 Stunden bei Raumtemperatur nachrühren. Das Reaktionsgemisch und auf Eiswasser gegossen und zweimal mit Methylenchlorid extrahiert. Die Extrakte werden mit Wasser gewaschen, über wasserfreiem Natriumsulfat getrocknet und unter vermindertem Druck eingedampft. Der ölige Rückstand wird an Kieselgel mit n-Hexan-Essigester (1:1) als Laufmittel chromatographiert. Man erhält den N-Methoxycarbonyl-5,7-dihydro-6H-dibenz[c,e]azepin -6-carboximidsäure-äthylester als Oel, Massenspektrum m/e: $M^+$ 324 (6), 295 (20), 263 (70), 194 (100), 178 (35), 165 (49).

In analoger Weise erhält man ausgehend von
- 5,7-Dihydro-6H-dibenz[c,e]azepin-6-carboximidsäure-äthylester und Chlorameisensäure-äthylester den N-Aethoxycarbonyl-5,7-dihydro-6H-dibenz[c,e]azepin-6-carboximidsäure-äthylester als Oel, Massenspektrum m/e: $M^+$ 338 (14), 309 (19), 293 (12), 263 (78), 194 (100);
- 5,7-Dihydro-6H-dibenz[c,e]azepin-6-carboximidsäure-äthylester und Chlorameisensäure-phenylester den N-Phenoxycarbonyl-5,7-dihydro-6H-dibenz[c,e]azepin-6-carboximidsaure-äthylester als Harz, Massenspektrum m/e: 293 (100, M-Phenolatradikal), 265 (16), 167 (76);
- 5,7-Dihydro-6H-dibenz[c,e]azepin-6-carboximidsäure-äthylester und Chlorameisensäure-allylester den N-Allyloxycarbonyl-5,7-dihydro-6H-dibenz[c,e]azepin-6-carboximidsäure-äthylester, Smp. 76-77,5° C;
- 5,7-Dihydro-6H-dibenz[c,e]azepin-6-carboximidsäure-äthylester und Chlorameisensäure-(2-chloräthyl)ester den N-(2-Chloräthoxycarbonyl)-5,7-dihydro-6H-dibenz[c,e]azepin-6-carboximidsäure-äthylester als Oel, Massenspektrum m/e: $M^+$ 372 (4), 343 (12), 293 (10), 263 (72), 194 (100), 178 (86), 165 (79);
- 5,7-Dihydro-6H-dibenz[c,e]azepin-6-carboximidsäure-äthylester und Chlorameisensäure-benzylester den N-Benzyloxycarbonyl-5,7-dihydro-6H-dibenz[c,e]azepin-6-carboximidsäure-äthylester als Sirup, Massenspektrum m/e: $M^+$ 400 (6), 371 (6), 309 (32), 263 (20), 194 (35), 179 (15), 167 (27), 108 (12), 91 (100);
- 5,7-Dihydro-6H-dibenz[c,e]azepin-6-carboximidsäure-äthylester und Chlorameisensäure-butylester den N-Butoxycarbonyl-5,7-dihydro-6H-dibenz[c,e]azepin-6-carboximidsäure-äthylester als Sirup, Massenspektrum m/e: $M^+$ 366 (6), 337 (10), 293 (12), 263 (67), 194 (100);
- 5,7-Dihydro-6H-dibenz[c,e]azepin-6-carboximidsäure-äthylester und Chlorameisensäure-isobutylester den N-Isobutoxycarbonyl-5,7-dihydro-6H-dibenz[c,e]azepin-6-carboximidsäure-äthylester als Sirup, Massenspektrum m/e: $M^+$ 366 (11), 337 (13), 293 (20), 263 (88), 194 (100);
- 5,7-Dihydro-6H-dibenz[c,e]azepin-6-carboximidsäure-äthylester und Chlorameisensäure-propylester den N-Propoxycarbonyl-5,7-dihydro-6H-dibenz[c,e]azepin-6-carboximidsäure-äthylester als Sirup, Massenspektrum m/e: $M^+$ 352 (8), 323 (14), 293 (14), 263 (78), 194 (100).

## Beispiel 6

2,0 g 5,7-Dihydro-6H-dibenz[c,e]azepin -6-carboximidsäure-äthylester werden in 10 ml Methylenchlorid vorgelegt. Nach Zugabe eines Tropfens Triäthylamin werden 0,41 g Methylisocyanat in 5 ml Methylenchlorid zugetropft. Dann lässt man 2 Tage bei Raumtemperatur nachrühren. Das Reaktionsgemisch wird auf Eiswasser gegossen und zweimal mit Methylenchlorid extrahiert. Die Extrakte werden mit Wasser gewaschen, über wasserfreiem Natriumsulfat getrocknet und unter vermindertem Druck eingedampft. Der ölige Rückstand wird an Kieselgel mit n-Hexan-Aceton (4:1) als Laufmittel chromatographiert. Man erhält den 5,7-Dihydro-N-methylcarbamoyl-6H-dibenz[c,e]azepin-6-carboximidsäure-äthylester in 2 isomeren Formen: Isomer A (zuerst eluiert), Smp. 176-178 °C, Isomer B: Smp. 137-139 °C.

In analoger Weise erhält man ausgehend von

- 5,7-Dihydro-6H-dibenz[c,e]azepin -6-carboximidsäure-äthylester und p-Chlorphenylisocyanat den N-[(p-Chlorphenyl)carbamoyl]-5,7-dihydro-6H-dibenz[c,e]azepin-6-carboximidsäure-äthylester, Smp. 154-156 °C;
- 5,7-Dihydro-6H-dibenz[c,e]azepin-6-carboximidsäure-äthylester und p-(Trifluormethoxy)-phenylisocyanat den N-{[p-(Trifluormethoxy)-phenyl]carbamoyl}-5,7-dihydro-6H-dibenz[c,e]azepin-6-carboximidsäure-äthylester. Smp. 129-130 °C.

## Beispiel 7

2,0 g 5,7-Dihydro-6H-dibenz[c,e]azepin-6-carboximidsäure-äthylester und 1,83 g 1,1′-Carbonyldiimidazol werden in 20 ml Tetrahydrofuran während 5 Stunden bei Raumtemperatur gerührt. Das Reaktionsgemisch wird auf Wasser gegossen und zweimal mit Essigester extrahiert.Die Extrakte werden mit gesättigter Kochsalzlösung gewaschen, über wasserfreiem Natriumsulfat getrocknet und eingedampft. Der Rückstand wird an Kieselgel mit n-Hexan-Aceton (1:4) als Laufmittel chromatographiert. Man erhält den N-(1-Imidazolylcarbonyl)-5,7-dihydro-6H-dibenz[c,e]azepin-6-carboximidsaure-äthylester, Smp. 134-137 °C.

## II. Formulierungsbeispiele:

## Beispiel 8

Ein Spritzpulver hat folgende Zusammensetzung:

| | Gewichtsprozent |
|---|---|
| Verbindung der Formel I bzw. Säureadditionssalz davon (Wirkstoff) | 50 |
| Hydratisierte Kieselsäure (Trägerstoff) | 37 |
| Polycarbonsäure-Natriumsalz (Dispergiermittel) | 4 |
| Nonylphenyl-(10)äthoxylat (Netzmittel) | 4 |
| Kaolin (Trägerstoff) | 5 |
| | 100 |

Der Wirkstoff wird mit dem Kaolin vermischt und separat das Netzmittel auf die hydratisierte Kieselsäure aufgezogen und das Dispergiermittel zugesetzt. Dann wird das Ganze homogen vermischt und in einer geeigneten Mühle feingemahlen. Das nun vorliegende Spritzpulver wird durch Wasser spontan benetzt und ergibt auf diese Weise eine gebrauchsfertige Dispersion.

## Beispiel 9

Ein emulgierbares Konzentrat hat folgende Zusammensetzung:

| | g/Liter |
|---|---|
| Verbindung der Formel I (Wirkstoff) | 250 |
| Polyarylphenol-(18)äthoxylat (Emulgator) | 300 |
| Isoterdecylalkohol (Antischaummittel) | 20 |
| Polyvinylpyrrolidon (Dispergiermittel) | 20 |
| N-Methyl-pyrrolidon (Lösungsmittel) | ad 1000 ml |

Der Wirkstoff, der Emulgator und das Antischaummittel werden unter Rühren in das Lösungsmittel aufgenommen. Hierauf wird das Dispergiermittel zugegeben und unter Rühren aufge löst. Nach Verdünnen mit Wasser ergibt das so entstandene emulgierbare Konzentrat eine Emulsion, die sich als gebrauchsfertige Spritzbrühe gut eignet.

## Ansprüche

1. Verbindungen der allgemeinen Formel

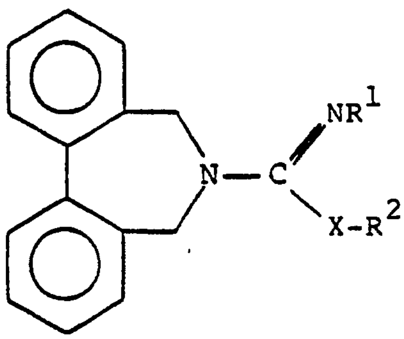

I

worin

$R^1$ Wasserstoff oder eine Gruppe $-Y-R^3$ ist,

$R^2$ mit $C_{3-6}$-Cycloalkyl, Aryl, Aryloxy oder Heteroaryl substituiertes $C_{1-6}$-Alkyl, $C_{3-6}$-Alkenyl oder $C_{3-6}$-Alkinyl ist, wobei im Falle von $R^1 = -Y-R^3$ die Substitution fakultativ ist,

oder

$R^2$ gegebenenfalls mit Aryl oder Aryloxy substituiertes, durch ein oder zwei Sauerstoffatome unterbrochenes $C_{2-6}$-Alkyl ist,

oder

$R^2$ Aryl, Heteroaryl, 2-Tetrahydrofuranylmethyl, 2-Tetrahydropyranylmethyl oder eine der Gruppen (a) bis (c)

$-N=CR^4R^5$ (a)

$-Z-ON=CR^4R^5$ (b)

$-Z-NR^6R^7$ (c)

ist,

$R^3$ Wasserstoff; gegebenenfalls substituiertes $C_{1-6}$-Alkyl, $C_{2-6}$-Alkenyl, $C_{2-6}$-Alkinyl, $C_{3-6}$-Cycloalkyl, Phenyl, Naphthyl, p-Biphenylyl, Benzylphenyl, Phenoxyphenyl, Benzoylphenyl, 3,4-Methylendioxyphenyl oder fünf- bis sechs-gliedriges Heterocyclyl; eine Gruppe $-OR^8$ (d); oder eine Gruppe $-NR^9R^{10}$ (e),

$R^4$ $C_{1-6}$-Alkyl,

$R^5$ $C_{1-6}$-Alkyl oder Phenyl,

oder

$R^4$ und $R^5$ zusammen Tetra-, Penta- oder Hexamethylen,

$R^6$ Wasserstoff oder $C_{1-4}$-Alkyl,

$R^7$ $C_{1-4}$-Alkyl, $C_{2-5}$-Alkanoyl oder $C_{2-5}$-Alkoxycarbonyl,

oder

$R^6$ und $R^7$ zusammen mit dem Stickstoffatom, an das sie gebunden sind, eine gegebenenfalls mit einem oder zwei $C_{1-4}$-Alkylresten substituierte fünf- bis siebengliedrige heterocyclische Gruppe, die neben

dem bereits erwähnten Stickstoffatom auch noch 1 oder 2 aus Sauerstoff, Schwefel und Stickstoff ausgewählte Heteroatome und/oder eine Ketofunktion im Ring aufweisen kann,

$R^8$ $C_{1-6}$-Alkyl, $C_{2-6}$-Halogenalkyl, $C_{3-6}$-Alkenyl, $C_{3-6}$-Alkinyl, Benzyl oder gegebenenfalls substituiertes Phenyl,

$R^9$ Wasserstoff oder $C_{1-4}$-Alkyl,

$R^{10}$ $C_{1-6}$-Alkyl oder gegebenenfalls substituiertes Phenyl,

X Sauerstoff oder Schwefel, wobei, falls $R^2$ eine Gruppe (a) ist, nur Sauerstoff,

Y Carbonyl, Sulfinyl oder Sulfonyl, wobei, falls $R^3$ Wasserstoff oder eine Gruppe (d) oder (e) ist, nur Carbonyl,

und

Z $C_{1-4}$-Alkylen bedeuten,

sowie Säureadditionssalze derjenigen Verbindungen I, in denen $R^1$ Wasserstoff bedeutet.

2. Verbindungen nach Anspruch 1, dadurch gekennzeichnet, dass $R^3$ Wasserstoff; gegebenenfalls substituiertes $C_{1-6}$-Alkyl, $C_{3-6}$-Alkenyl, $C_{3-6}$-Alkinyl, $C_{3-6}$-Cycloalkyl, Phenyl, Naphthyl, p-Biphenyl, Benzylphenyl, Phenoxyphenyl, Benzoylphenyl oder fünf- bis sechs-gliedriges Heterocyclyl; eine Gruppe -$OR^8$ - (d), in der $C_{1-6}$-Alkyl oder gegebenenfalls substituiertes Phenyl ist; oder eine Gruppe -$NR^9R^{10}$ (e), in der $R^9$ und $R^{10}$ die in Anspruch 1 angegebenen Bedeutungen besitzen, bedeutet.

3. Verbindungen nach Anspruch 1 oder 2, dadurch gekennzeichnet, dass $R^1$ eine Gruppe -Y-$R^3$ bedeutet.

4. Verbindungen nach einem der Ansprüche 1 bis 3, dadurch gekennzeichnet, dass X Sauerstoff bedeutet.

5. Verbindungen nach Anspruch 1, dadurch gekennzeichnet, dass $R^1$ Wasserstoff, $R^2$ eine Gruppe (b) oder (c) und Z Aethylen bedeuten.

6. Verbindungen nach Anspruch 3, dadurch gekennzeichnet, dass $R^2$ $C_{1-4}$-Alkyl, $R^3$ gegebenenfalls substituiertes Phenyl, gegebenenfalls substituiertes fünf- bis sechs-gliedriges Heterocyclyl oder eine Gruppe (d) und Y Carbonyl bedeuten.

7. Eine Verbindung nach Anspruch 1, ausgewählt aus:

5,7-Dihydro-6H-dibenz[c,e]azepin-6-carboximidsäure-{2-[(isopropylidenamino)oxy]-äthyl}ester,
5,7-Dihydro-6H-dibenz[c,e]azepin-6-carboximidsaure-(2-äthoxycarbonylamino-äthyl)ester,
N-Benzoyl-5,7-dihydro-6H-dibenz[c,e]azepin-6-carboximidsäure-äthylester,
N-(p-Chlorbenzoyl)-5,7-dihydro-6H-dibenz[c,e]azepin-6-carboximidsäure-äthylester,
N-Isonicotinoyl-5,7-dihydro-6H-dibenz[c,e]azepin-6-carboximidsäure-äthylester,
N-Aethoxycarbonyl-5,7-dihydro-6H-dibenz[c,e]azepin-6-carboximidsäure-äthylester,
N-(1-Imidazolylcarbonyl)-5,7-dihydro-6H-dibenz[c,e]azepin-6-carboximidsäure-äthylester,
N-(p-Brombenzoyl)-5,7-dihydro-6H-dibenz[c,e]azepin-6-carboximidsäure-äthylester,
N-(p-tert.Butylbenzoyl)-5,7-dihydro-6H-dibenz[c,e]azepin-6-carboximidsäure-äthylester,
N-(p-Fluorbenzoyl)-5,7-dihydro-6H-dibenz[c,e]azepin-6-carboximidsäure-äthylester,
N-Methoxycarbonyl-5,7-dihydro-6H-dibenz[c,e]azepin-6-carboximidsäure-äthylester,
5,7-Dihydro-6H-dibenz[c,e]azepin-6-carboximidsäure-cyclopropylmethylester,
N-(p-Trifluormethylbenzoyl)-5,7-dihydro-6H-dibenz[c,e]azepin-6-carboximidsäure-äthylester,
N-(p-Anisoyl)-5,7-dihydro-6H-dibenz[c,e]azepin-6-carboximidsäure-äthylester,
N-Benzoyl-5,7-dihydro-6H-dibenz[c,e]azepin-6-carboximidsäure-methylester,
N-Benzoyl-5,7-dihydro-6H-dibenz[c,e]azepin-6-carboximidsäure-propylester,
N-(2-Furoyl)-5,7-dihydro-6H-dibenz[c,e]azepin-6-carboximidsäure-äthylester und
N-Cyclopropylcarbonyl-5,7-dihydro-6H-dibenz[c,e]azepin-6-carboximidsäure-äthylester.

8. Schädlingsbekämpfungsmittel, dadurch gekennzeichnet, dass es eine wirksame Menge mindestens einer Verbindung der allgemeinen Formel

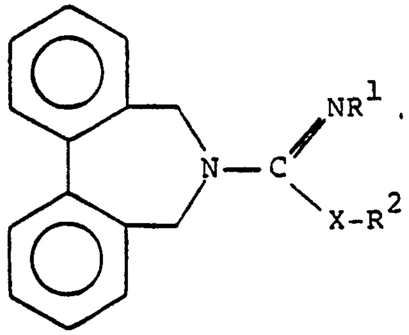

I

worin
$R^1$ Wasserstoff oder eine Gruppe -Y-$R^3$ ist,
$R^2$ mit $C_{3-6}$-Cycloalkyl, Aryl, Aryloxy oder Heteroaryl substituiertes $C_{1-6}$-Alkyl, $C_{3-6}$-Alkenyl oder $C_{3-6}$-Alkinyl ist, wobei im Falle von $R^1$ = -Y-$R^3$ die Substitution fakultativ ist,
oder
$R^2$ gegebenenfalls mit Aryl oder Aryloxy substituiertes, durch ein oder zwei Sauerstoffatome unterbrochenes $C_{2-6}$-Alkyl ist,
oder
$R^2$ Aryl, Heteroaryl, 2-Tetrahydrofuranylmethyl, 2-Tetrahydropyranylmethyl oder eine der Gruppen (a) bis (c)
-N=$CR^4R^5$ (a)
-Z-ON=$CR^4R^5$ (b)
-Z-$NR^6R^7$ (c)
ist,
$R^3$ Wasserstoff; gegebenenfalls substituiertes $C_{1-6}$-Alkyl, $C_{2-6}$-Alkenyl, $C_{2-6}$-Alkinyl, $C_{3-6}$-Cycloalkyl, Phenyl, Naphthyl, p-Biphenylyl, Benzylphenyl, Phenoxyphenyl, Benzoylphenyl, 3,4-Methylendioxyphenyl oder fünf- bis sechs-glie driges Heterocyclyl; eine Gruppe -$OR^8$ (d); oder eine Gruppe -$NR^9R^{10}$ (e),
$R^4$ $C_{1-6}$-Alkyl,
$R^5$ $C_{1-6}$-Alkyl oder Phenyl,
oder
$R^4$ und $R^5$ zusammen Tetra-, Penta- oder Hexamethylen,
$R^6$ Wasserstoff oder $C_{1-4}$-Alkyl,
$R^7$ $C_{1-4}$-Alkyl, $C_{2-5}$-Alkanoyl oder $C_{2-5}$-Alkoxycarbonyl,
oder
$R^6$ und $R^7$ zusammen mit dem Stickstoffatom, an das sie gebunden sind, eine gegebenenfalls mit einem oder zwei $C_{1-4}$-Alkylresten substituierte fünf- bis siebengliedrige heterocyclische Gruppe, die neben dem bereits erwähnten Stickstoffatom auch noch 1 oder 2 aus Sauerstoff, Schwefel und Stickstoff ausgewählte Heteroatome und/oder eine Ketofunktion im Ring aufweisen kann,
$R^8$ $C_{1-6}$-Alkyl, $C_{2-6}$-Halogenalkyl, $C_{3-6}$-Alkenyl, $C_{3-6}$-Alkinyl, Benzyl oder gegebenenfalls substituiertes Phenyl,
$R^9$ Wasserstoff oder $C_{1-4}$-Alkyl,
$R^{10}$ $C_{1-6}$-Alkyl oder gegebenenfalls substituiertes Phenyl,
X Sauerstoff oder Schwefel, wobei, falls $R^2$ eine Gruppe (a) ist, nur Sauerstoff,
Y Carbonyl, Sulfinyl oder Sulfonyl, wobei, falls $R^3$ Wasserstoff oder eine Gruppe (d) oder (e) ist, nur Carbonyl,
und
Z $C_{1-4}$-Alkylen bedeuten,
oder eines Säureadditionssalzes einer solchen Verbindung, in der $R^1$ Wasserstoff bedeutet,
sowie Formulierungshilfsstoffe enthält.

9. Schädlingsbekämpfungsmittel nach Anspruch 8, dadurch gekennzeichnet, dass es eine wirksame Menge mindestens einer aus der Gruppe
5,7-Dihydro-6H-dibenz[c,e]azepin-6-carboximidsäure-{2-[(isopropylidenamino)oxy]-äthyl}ester,
5,7-Dihydro-6H-dibenz[c,e]azepin-6-carboximidsäure-(2-äthoxycarbonylamino-äthyl)ester,
N-Benzoyl-5,7-dihydro-6H-dibenz[c,e]azepin-6-carboximidsäure-äthylester,
N-(p-Chlorbenzoyl)-5,7-dihydro-6H-dibenz[c,e]azepin-6-carboximidsäure-äthylester,

N-Isonicotinoyl-5,7-dihydro-6H-dibenz[c,e]azepin-6-carboximidsäure-äthylester,
N-Aethoxycarbonyl-5,7-dihydro-6H-dibenz[c,e]azepin-6-carboximidsäure-äthylester,
N-(1-Imidazolylcarbonyl)-5,7-dihydro-6H-dibenz[c,e]azepin-6-carboximidsäure-äthylester,
N-(p-Brombenzoyl)-5,7-dihydro-6H-dibenz[c,e]azepin-6-carboximidsäure-äthylester,
N-(p-tert.Butylbenzoyl)-5,7-dihydro-6H-dibenz[c,e]azepin-6-carboximidsäure-äthylester,
N-(p-Fluorbenzoyl)-5,7-dihydro-6H-dibenz[c,e]azepin-6-carboximidsäure-äthylester,
N-Methoxycarbonyl-5,7-dihydro 6H-dibenz[c,e]azepin-6-carboximidsäure-äthylester,
5,7-Dihydro-6H-dibenz[c,e]azepin-6-carboximidsäure-cyclopropylmethylester,
N-(p-Trifluormethylbenzoyl)-5,7-dihydro-6H-dibenz[c,e]azepin-6-carboximidsäure-äthylester,
N-(p-Anisoyl)-5,7-dihydro-6H-dibenz[c,e]azepin-6-carboximidsäure-äthylester,
N-Benzoyl-5,7-dihydro-6H-dibenz[c,e]azepin-6-carboximidsäure-methylester,
N-Benzoyl-5,7-dihydro-6H-dibenz[c,e]azepin-6-carboximidsäure-propylester,
N-(2-Furoyl)-5,7-dihydro-6H-dibenz[c,e]azepin-6-carboximidsäure-äthylester und
N-Cyclopropylcarbonyl-5,7-dihydro-6H-dibenz[c,e]azepin-6-carboximidsäure-äthylester
ausgewählten Verbindung sowie Formulierungshilfsstoffe enthält.

10. Verfahren zur Herstellung von Verbindungen der allgemeinen Formel

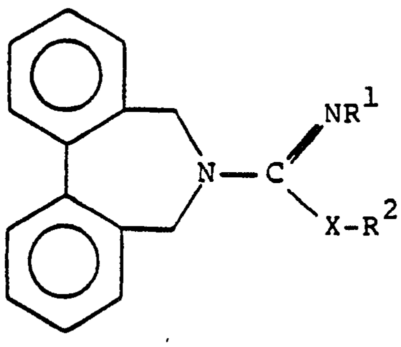

I

worin

$R^1$ Wasserstoff oder eine Gruppe -Y-$R^3$ ist,

$R^2$ mit $C_{3-6}$-Cycloalkyl, Aryl, Aryloxy oder Heteroaryl substituiertes $C_{1-6}$-Alkyl, $C_{3-6}$-Alkenyl oder $C_{3-6}$-Alkinyl ist, wobei im Falle von $R^1$ = -Y-$R^3$ die Substitution fakultativ ist,

oder

$R^2$ gegebenenfalls mit Aryl oder Aryloxy substituiertes, durch ein oder zwei Sauerstoffatome unterbrochenes $C_{2-6}$-Alkyl ist,

oder

$R^2$ Aryl, Heteroaryl, 2-Tetrahydrofuranylmethyl, 2-Tetrahydropyranylmethyl oder eine der Gruppen (a) bis (c)

-N = $CR^4R^5$ (a)

-Z-ON = $CR^4R^5$ (b)

-Z-$NR^6R^7$ (c)

ist,

$R^3$ Wasserstoff; gegebenenfalls substituiertes $C_{1-6}$-Alkyl, $C_{2-6}$-Alkenyl, $C_{2-6}$-Alkinyl, $C_{3-6}$-Cycloalkyl, Phenyl, Naphthyl, p-Biphenylyl, Benzylphenyl, Phenoxyphenyl, Benzoylphenyl, 3,4-Methylendioxyphenyl oder fünf- bis sechs-gliedriges Heterocyclyl; eine Gruppe -$OR^8$ (d); oder eine Gruppe $NR^9R^{10}$ (e),

$R^4$ $C_{1-6}$-Alkyl,

$R^5$ $C_{1-6}$-Alkyl oder Phenyl,

oder

$R^4$ und $R^5$ zusammen Tetra-, Penta- oder Hexamethylen,

$R^6$ Wasserstoff oder $C_{1-4}$-Alkyl,

$R^7$ $C_{1-4}$-Alkyl, $C_{2-5}$-Alkanoyl oder $C_{2-5}$-Alkoxycarbonyl,

oder

$R^6$ und $R^7$ zusammen mit dem Stickstoffatom, an das sie gebunden sind, eine gegebenenfalls mit einem oder zwei $C_{1-4}$-Alkylresten substituierte fünf- bis siebengliedrige heterocyclische Gruppe, die neben dem bereits erwähnten Stickstoffatom auch noch 1 oder 2 aus Sauerstoff, Schwefel und Stickstoff ausgewählte Heteroatome und/oder eine Ketofunktion im Ring aufweisen kann,

$R^8$ $C_{1-6}$-Alkyl, $C_{2-6}$-Halogenalkyl, $C_{3-6}$-Alkenyl, $C_{3-6}$-Alkinyl, Benzyl oder gegebenenfalls substituiertes Phenyl,
$R^9$ Wasserstoff oder $C_{1-4}$-Alkyl,
$R^{10}$ $C_{1-6}$-Alkyl oder gegebenenfalls substituiertes Phenyl,
X Sauerstoff oder Schwefel, wobei, falls $R^2$ eine Gruppe (a) ist, nur Sauerstoff,
Y Carbonyl, Sulfinyl oder Sulfonyl, wobei, falls $R^3$ Wasserstoff oder eine Gruppe (d) oder (e) ist, nur Carbonyl,
und
Z $C_{1-4}$-Alkylen bedeuten,
und von den Säureadditionssalzen derjenigen Verbindungen I, in denen $R^1$ Wasserstoff bedeutet, dadurch gekennzeichnet, dass man
a) zwecks Herstellung derjenigen Verbindungen der Formel I, in denen $R^1$ Wasserstoff bedeutet, 5,7-Dihydro-6H-dibenz[c,e]azepin -6-carbonitril, d.h. das Cyanamid der Formel

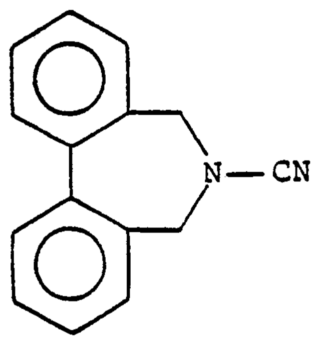
II

mit einem Alkohol bzw. Thiol der allgemeinen Formel
$R^{2'}$-$X'$H III
worin
$R^{2'}$ und $X'$ diejenigen oben angegebenen Bedeutungen von $R^2$ und X besitzen, die für den Zustand gelten, dass $R^1$ für Wasserstoff steht,
oder mit einem Alkalimetallsalz davon umsetzt, oder
b) zwecks Herstellung derjenigen Verbindungen der Formel I, in denen $R^1$ eine Gruppe -Y-$R^3$ bedeutet, ein Carboximidsäureester der allgemeinen Formel

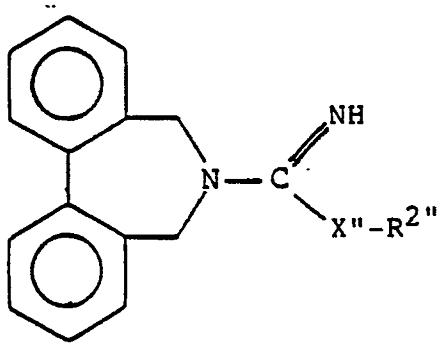
IV

worin
$R^{2''}$ und $X''$ diejenigen oben angegebenen Bedeutungen von $R^2$ und X besitzen, die für den Zustand gelten, dass $R^1$ für -Y-$R^3$ steht,
mit einer Carbonyl-, Sulfinyl- oder Sulfonylverbindung der allgemeinen Formel
Q-Y-$R^3$ V
worin
$R^3$ und Y die oben angegebenen Bedeutungen besitzen
und
Q eine Abgangsgruppe bedeutet,

umsetzt
und erwünschtenfalls eine erhaltene Verbindung der Formel I, in der $R^1$ Wasserstoff bedeutet, durch Umsetzung mit einer Säure in das entsprechende Säureadditionssalz überführt.

11. Verfahren zur Bekämpfung von Schädlingen, dadurch gekennzeichnet, dass man das zu schützende Gut oder die Schädlinge selbst mit einer wirksamen Menge einer Verbindung gemäss einem der Ansprüche 1 bis 7 bzw. eines Mittels gemäss Anspruch 8 oder 9 behandelt.

12. Verwendung einer Verbindung gemäss einem der Ansprüche 1 bis 7 bzw. eines Mittels gemäss Anspruch 8 oder 9 zur Bekämpfung von Schädlingen.

Patentansprüche für folgenden Vertragsstaat: ES

10. Verfahren zur Herstellung von Verbindungen der allgemeinen Formel

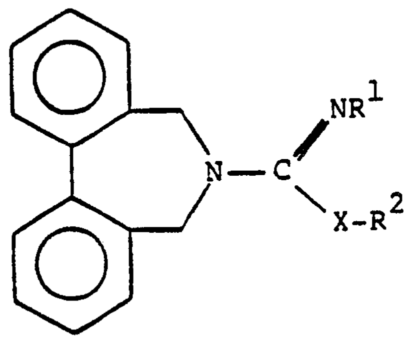

I

worin
$R^1$ Wasserstoff oder eine Gruppe -Y-$R^3$ ist,
$R^2$ mit $C_{3-6}$-Cycloalkyl, Aryl, Aryloxy oder Heteroaryl substituiertes $C_{1-6}$-Alkyl, $C_{3-6}$-Alkenyl oder $C_{3-6}$-Alkinyl ist, wobei im Falle von $R^1$ = -Y-$R^3$ die Substitution fakultativ ist,
oder
$R^2$ gegebenenfalls mit Aryl oder Aryloxy substituiertes, durch ein oder zwei Sauerstoffatome unterbrochenes $C_{2-6}$-Alkyl ist,
oder
$R^2$ Aryl, Heteroaryl, 2-Tetrahydrofuranylmethyl, 2-Tetrahydropyranylmethyl oder eine der Gruppen (a) bis (c)
-N = $CR^4R^5$ (a)
-Z-ON = $CR^4R^5$ (b)
-Z-$NR^6R^7$ (c)
ist,
$R^3$ Wasserstoff; gegebenenfalls substituiertes $C_{1-6}$-Alkyl, $C_{2-6}$-Alkenyl, $C_{2-6}$-Alkinyl, $C_{3-6}$-Cycloalkyl, Phenyl, Naphthyl, p-Biphenylyl, Benzylphenyl, Phenoxyphenyl, Benzoylphenyl, 3,4-Methylendioxyphenyl oder fünf- bis sechs-gliedriges Heterocyclyl; eine Gruppe -$OR^8$ (d); oder eine Gruppe -$NR^9R^{10}$ e),
$R^4$ $C_{1-6}$-Alkyl,
$R^5$ $C_{1-6}$-Alkyl oder Phenyl,
oder
$R^4$ und $R^5$ zusammen Tetra-, Penta- oder Hexamethylen,
$R^6$ Wasserstoff oder $C_{1-4}$-Alkyl,
$R^7$ $C_{1-4}$-Alkyl, $C_{2-5}$-Alkanoyl oder $C_{2-5}$-Alkoxycarbonyl,
oder
$R^6$ und $R^7$ zusammen mit dem Stickstoffatom, an das sie gebunden sind, eine gegebenenfalls mit einem oder zwei $C_{1-4}$-Alkylresten substituierte fünf- bis siebengliedrige heterocyclische Gruppe, die neben dem bereits erwähnten Stickstoffatom auch noch 1 oder 2 aus Sauerstoff, Schwefel und Stickstoff ausgewählte Heteroatome und/oder eine Ketofunktion im Ring aufweisen kann,
$R^8$ $C_{1-6}$-Alkyl, $C_{2-6}$-Halogenalkyl, $C_{3-6}$-Alkenyl, $C_{3-6}$-Alkinyl, Benzyl oder gegebenenfalls substituiertes Phenyl,
$R^9$ Wasserstoff oder $C_{1-4}$-Alkyl,
$R^{10}$ $C_{1-6}$-Alkyl oder gegebenenfalls substituiertes Phenyl,

X Sauerstoff oder Schwefel, wobei, falls $R^2$ eine Gruppe (a) ist, nur Sauerstoff,
Y Carbonyl, Sulfinyl oder Sulfonyl, wobei, falls $R^3$ Wasserstoff oder eine Gruppe (d) oder (e) ist, nur Carbonyl,
und
Z $C_{1-4}$-Alkylen bedeuten,
und von den Säureadditionssalzen derjenigen Verbindungen I, in denen $R^1$ Wasserstoff bedeutet, dadurch gekennzeichnet, dass man
a) zwecks Herstellung derjenigen Verbindungen der Formel I, in denen $R^1$ Wasserstoff bedeutet, 5,7-Dihydro-6H-dibenz[c,e]azepin -6-carbonitril, d.h. das Cyanamid der Formel

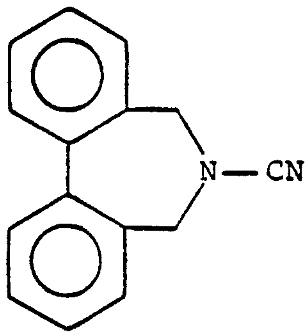

II

mit einem Alkohol bzw. Thiol der allgemeinen Formel
$R^2$-$X'$H III
worin
$R^2$ und $X'$ diejenigen oben angegebenen Bedeutungen von $R^2$ und X besitzen, die für den Zustand gelten, dass $R^1$ für Wasserstoff steht,
oder mit einem Alkalimetallsalz davon umsetzt, oder
b) zwecks Herstellung derjenigen Verbindungen der Formel I, in denen $R^1$ eine Gruppe -Y-$R^3$ bedeutet, ein Carboximidsäureester der allgemeinen Formel

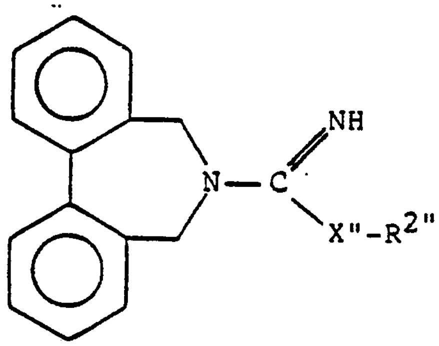

IV

worin
$R^2$ und $X''$ diejenigen oben angegebenen Bedeutungen von $R^2$ und X besitzen, die für den Zustand gelten, dass $R^1$ für -Y-$R^3$ steht,
mit einer Carbonyl-, Sulfinyl- oder Sulfonylverbindung der allgemeinen Formel
Q-Y-$R^3$ V
worin
$R^3$ und Y die oben angegebenen Bedeutungen besitzen
und
Q eine Abgangsgruppe bedeutet,
umsetzt
und erwünschtenfalls eine erhaltene Verbindung der Formel I, in der $R^1$ Wasserstoff bedeutet, durch Umsetzung mit einer Säure in das entsprechende Säureadditionssalz überführt.

2. Verfahren nach Anspruch 1, dadurch gekennzeichnet, dass $R^3$ Wasserstoff; gegebenenfalls substitu-

iertes $C_{1-6}$-Alkyl, $C_{3-6}$-Alkenyl, $C_{3-6}$-Alkinyl, $C_{3-6}$-Cycloalkyl, Phenyl, Naphthyl, p-Biphenyl, Benzylphenyl, Phenoxyphenyl, Benzoylphenyl oder fünf- bis sechs-gliedriges Heterocyclyl; eine Gruppe $-OR^8$ (d), in der $R^8$ $C_{1-6}$-Alkyl oder gegebenenfalls substituiertes Phenyl ist; oder eine Gruppe $-NR^9R^{10}$ (e), in der $R^9$ und $R^{10}$ die in Anspruch 1 angegebenen Bedeutungen besitzen, bedeutet.

3. Verfahren nach Anspruch 1 oder 2, dadurch gekennzeichnet, dass $R^1$ eine Gruppe $-Y-R^3$ bedeutet.

4. Verfahren nach einem der Ansprüche 1 bis 3, dadurch gekennzeichnet, dass X Sauerstoff bedeutet.

5. Verfahren nach Anspruch 1, dadurch gekennzeichnet, dass $R^1$ Wasserstoff, $R^2$ eine Gruppe (b) oder (c) und Z Aethylen bedeuten.

6. Verfahren nach Anspruch 3, dadurch gekennzeichnet, dass $R^2$ $C_{1-4}$-Alkyl, $R^3$ gegebenenfalls substituiertes Phenyl, gegebenenfalls substituiertes fünf- bis sechs-gliedriges Heterocyclyl oder eine Gruppe (d) und Y Carbonyl bedeuten.

7. Verfahren nach Anspruch 1 zur Herstellung einer aus der Gruppe
5,7-Dihydro-6H-dibenz[c,e]azepin-6-carboximidsäure-{2-[(isopropylidenamino)oxy]-äthyl}ester,
5,7-Dihydro-6H-dibenz[c,e]azepin-6-carboximidsäure-(2-äthoxycarbonylamino-äthyl)ester,
N-Benzoyl-5,7-dihydro-6H-dibenz[c,e]azepin-6-carboximidsäure-äthylester,
N-(p-Chlorbenzoyl)-5,7-dihydro-6H-dibenz[c,e]azepin-6-carboximidsäure-athylester,
N-Isonicotinoyl-5,7-dihydro-6H-dibenz[c,e]azepin-6-carboximidsäure-äthylester,
N-Aethoxycarbonyl-5,7-dihydro-6H-dibenz[c,e]azepin-6-carboximidsäure-äthylester,
N-(1-Imidazolylcarbonyl)-5,7-dihydro-6H-dibenz[c,e]azepin-6-carboximidsäure-äthylester,
N-(p-Brombenzoyl)-5,7-dihydro-6H-dibenz[c,e]azepin-6-carboximidsäure-äthylester,
N-(p-tert.Butylbenzoyl)-5,7-dihydro-6H-dibenz[c,e]azepin-6-carboximidsäure-äthylester,
N-(p-Fluorbenzoyl)-5,7-dihydro-6H-dibenz[c,e]azepin-6-carboximidsäure-äthylester,
N-Methoxycarbonyl-5,7-dihydro-6H-dibenz[c,e]azepin-6-carboximidsäure-äthylester,
5,7-Dihydro-6H-dibenz[c,e]azepin-6-carboximidsäure-cyclopropylmethylester,
N-(p-Trifluormethylbenzoyl)-5,7-dihydro-6H-dibenz[c,e]azepin-6-carboximidsäure-äthylester,
N-(p-Anisoyl)-5,7-dihydro-6H-dibenz[c,e]azepin-6-carboximidsäure-äthylester,
N-Benzoyl-5,7-dihydro-6H-dibenz[c,e]azepin-6-carboximidsäure-methylester,
N-Benzoyl-5,7-dihydro-6H-dibenz[c,e]azepin-6-carboximidsäure-propylester,
N-(2-Furoyl)-5,7-dihydro-6H-dibenz[c,e]azepin-6-carboximidsäure-äthylester und
N-Cyclopropylcarbonyl-5,7-dihydro-6H-dibenz[c,e]azepin-6-carboximidsäure-äthylester
ausgewählten Verbindung.

8. Vefahren nach einem der Ansprüche 1 bis 7, dadurch gekennzeichnet, dass die Umsetzung gemäss der Verfahrensvariante a) unter Verwendung überschüssigen Alkohols bzw. Thiols der Formel III als Lösungsmittel, eventuell auch noch eines Hilfslösungsmittels, und in Gegenwart einer katalytischen oder stöchiometrischen Menge eines Alkalimetallsalzes des Alkohols bzw. Thiols der Formel III oder eines Alkalimetallcyanids, bei Temperaturen von 10 bis 120 °C, und dass die Umsetzung gemäss der Verfahrensvariante b) in Gegenwart eines inerten organischen Verdünnungsmittels und eines säurebindenden Mittels bei Temperaturen zwischen -20 °C und 100 °C erfolgt.

9. Schädlingsbekämpfungsmittel, dadurch gekennzeichnet, dass es eine wirksame Menge mindestens einer Verbindung der allgemeinen Formel

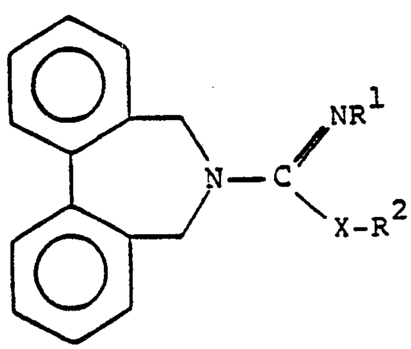

I

worin
$R^1$ Wasserstoff oder eine Gruppe $Y-R^3$ ist,
$R^2$ mit $C_{3-6}$-Cycloalkyl, Aryl, Aryloxy oder Heteroaryl substituiertes $C_{1-6}$-Alkyl, $C_{3-6}$-Alkenyl oder $C_{3-6}$-Alkinyl ist, wobei im Falle von $R^1$ = $-Y-R^3$ die Substitution fakultativ ist,

oder
$R^2$ gegebenenfalls mit Aryl oder Aryloxy substituiertes, durch ein oder zwei Sauerstoffatome unterbrochenes $C_{2-6}$-Alkyl ist,
oder
$R^2$ Aryl, Heteroaryl, 2-Tetrahydrofuranylmethyl, 2-Tetrahydropyranylmethyl oder eine der Gruppen (a) bis (c)
$-N=CR^4R^5$ (a)
$-Z-ON=CR^4R^5$ (b)
$-Z-NR^6R^7$ (c)
ist,
$R^3$ Wasserstoff; gegebenenfalls substituiertes $C_{1-6}$-Alkyl, $C_{2-6}$-Alkenyl, $C_{2-6}$-Alkinyl, $C_{3-6}$-Cycloalkyl, Phenyl, Naphthyl, p-Biphenylyl, Benzylphenyl, Phenoxyphenyl, Benzoylphenyl, 3,4-Methylendioxyphenyl oder fünf- bis sechs-gliedriges Heterocyclyl; eine Gruppe $-OR^8$ (d); oder eine Gruppe $-NR^9R^{10}$ (e),
$R^4$ $C_{1-6}$-Alkyl,
$R^5$ $C_{1-6}$-Alkyl oder Phenyl,
oder
$R^4$ und $R^5$ zusammen Tetra-, Penta- oder Hexamethylen,
$R^6$ Wasserstoff oder $C_{1-4}$-Alkyl,
$R^7$ $C_{1-4}$-Alkyl, $C_{2-5}$-Alkanoyl oder $C_{2-5}$-Alkoxycarbonyl,
oder
$R^6$ und $R^7$ zusammen mit dem Stiokstoffatom, an das sie gebunden sind, eine gegebenenfalls mit einem oder zwei $C_{1-4}$-Alkylresten substituierte fünf- bis siebengliedrige heterooyclische Gruppe, die neben dem bereits erwähnten Stickstoffatom auch noch 1 oder 2 aus Sauerstoff, Schwefel und Stickstoff ausgewählte Heteroatome und/oder eine Ketofunktion im Ring aufweisen kann,
$R^8$ $C_{1-6}$-Alkyl, $C_{2-6}$-Halogenalkyl, $C_{3-6}$-Alkenyl, $C_{3-6}$-Alkinyl, Benzyl oder gegebenenfalls substituiertes Phenyl,
$R^9$ Wasserstoff oder $C_{1-4}$-Alkyl,
$R^{10}$ $C_{1-6}$-Alkyl oder gegebenenfalls substituiertes Phenyl,
X Sauerstoff oder Schwefel, wobei, falls $R^2$ eine Gruppe (a) ist, nur Sauerstoff,
Y Carbonyl, Sulfinyl oder Sulfonyl, wobei, falls $R^3$ Wasserstoff oder eine Gruppe (d) oder (e) ist, nur Carbonyl,
und
Z $C_{1-4}$-Alkylen bedeuten,
oder eines Säureadditionssalzes einer solchen Verbindung, in der $R^1$ Wasserstoff bedeutet, sowie Formulierungshilfsstoffe enthält.

10. Schädlingsbekämpfungsmittel nach Anspruch 9, dadurch gekennzeichnet, dass es eine wirksame Menge mindestens einer aus der Gruppe
5,7-Dihydro-6H-dibenz[c,e]azepin-6-carboximidsäure-{2-[(isopropylidenamino)oxy]-äthyl}ester,
5,7-Dihydro-6H-dibenz[c,e]azepin-6-carboximidsäure-(2-äthoxycarbonylamino-äthyl)ester,
N-Benzoyl-5,7-dihydro-6H-dibenz[c,e]azepin-6-carboximidsäure-äthylester,
N-(p-Chlorbenzoyl)-5,7-dihydro-6H-dibenz[c,e]azepin-6-carboximidsäure-äthylester,
N-Isonicotinoyl-5,7-dihydro-6H-dibenz[c,e]azepin-6-carboximidsäure-äthylester,
N-Aethoxycarbonyl-5,7-dihydro-6H-dibenz[c,e]azepin-6-carboximidsäure-äthylester,
N-(1-Imidazolylcarbonyl)-5,7-dihydro-6H-dibenz[c,e]azepin-6-carboximidsäure-äthylester,
N-(p-Brombenzoyl)-5,7-dihydro-6H-dibenz[c,e]azepin-6-carboximidsäure-äthylester,
N-(p-tert.Butylbenzoyl)-5,7-dihydro-6H-dibenz[c,e]azepin-6-carboximidsäure-äthylester,
N-(p-Fluorbenzoyl)-5,7-dihydro-6H-dibenz[c,e]azepin-6-carboximidsäure-äthylester,
N-Methoxycarbonyl-5,7-dihydro-6H-dibenz[c,e]azepin-6-carboximidsäure-äthylester,
5,7-Dihydro-6H-dibenz[c,e]azepin-6-carboximidsäure-cyclopropylmethylester,
N-(p-Trifluormethylbenzoyl)-5,7-dihydro-6H-dibenz[c,e]azepin-6-carboximidsäure-äthylester,
N-(p-Anisoyl)-5,7-dihydro-6H-dibenz[c,e]azepin-6-carboximidsäure-äthylester,
N-Benzoyl-5,7-dihydro-6H-dibenz[c,e]azepin-6-carboximidsäure-methylester,
N-Benzoyl-5,7-dihydro-6H-dibenz[c,e]azepin-6-carboximidsäure-propylester,
N-(2-Furoyl)-5,7-dihydro-6H-dibenz[c,e]azepin-6-carboximidsäure-äthylester und
N-Cyclopropylcarbonyl-5,7-dihydro-6H-dibenz[c,e]azepin-6-carboximidsäure-äthylester
ausgewählten Verbindung sowie Formulierungshilfsstoffe enthält.

11. Verfahren zur Herstellung eines Schädlingsbekämpfungsmittels gemäss Anspruch 9, dadurch gekennzeichnet, dass man mindestens eine Verbindung der allgemeinen Formel I mit Formulierungshilfsstoffen vermischt.

12. Verfahren zur Bekämpfung von Schädlingen, dadurch gekennzeichnet, dass man das zu schützende Gut oder die Schädlinge selbst mit einer wirksamen Menge eines Mittels gemäss Anspruch 9 oder 10 behandelt.

Europäisches Patentamt

# EUROPÄISCHER RECHERCHENBERICHT

Nummer der Anmeldung

EP 89 12 0426

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | EP-A-0 192 034 (F. HOFFMANN-LA ROCHE UND CO. AG) * Ansprüche 1,3,12,14; Spalte 2, Zeile 49 - Spalte 4, Zeile 55; Spalten 3,4; Verbindung II * | 1,4,8, 10 | C 07 D 223/18<br>A 01 N 47/42 |
| | | | **RECHERCHIERTE SACHGEBIETE (Int. Cl.5)** |
| | | | A 01 N 47/00<br>C 07 D 223/00 |

Der vorliegende Recherchenbericht wurde für alle Patentansprüche erstellt

| Recherchenort | Abschlußdatum der Recherche | Prüfer |
|---|---|---|
| BERLIN | 16-01-1990 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)